# NEW EUROPEAN PATENT SPECIFICATION

(11) **EP 2 768 944 B2**
(45) Date of publication and mention of the opposition decision: **22.11.2023**
(45) Mention of the grant of the patent: 26.02.2020
(21) Application number: 12798373.2
(22) Date of filing: 09.10.2012
(51) Int. Cl.: C12P 21/00, C12N 5/071, C12N 5/00

(54) **ADDITION OF IRON TO IMPROVE CELL CULTURE**
ZUGABE VON EISEN ZUR VERBESSERUNG DER ZELLKULTUR
ADDITION DE FER POUR AMÉLIORER LA CULTURE CELLULAIRE

(30) Priority: 21.10.2011 US 201161550058 P
(43) Date of publication of application: 27.08.2014
(73) Proprietor: Pfizer Inc., New York, NY 10001-2192 (US)
(72) Inventor: WANG, Wenge, Andover, Massachusetts 01810 (US); LUAN, Yen-Tung, Chelmsford, Massachusetts 01824 (US); DRAPEAU, Denis, Windham, New Hampshire 03087 (US); NOLAN, Ryan P., Stoneham, Massachusetts 02180 (US)
(74) Representative: Pfizer
(86) International application number: PCT/IB2012/055457
(87) International publication number: WO 2013/057628

(56) References cited:
- WO-A1-98/08934
- WO-A1-2009/087087
- DAVID C. COHEN ET AL: "Biotin and choline replace the growth requirement of Madin-Darby canine kidney cells for high-density lipoproteins", JOURNAL OF CELLULAR PHYSIOLOGY, vol. 124, no. 1, 1 July 1985 (1985-07-01), pages 96-106, XP055054198, ISSN: 0021-9541, DOI: 10.1002/jcp.1041240116
- G. LESCOAT ET AL: "Antiproliferative and apoptotic effects in rat and human hepatoma cell cultures of the orally active iron chelator ICL670 compared to CP20: a possible relationship with polyamine metabolism", CELL PROLIFERATION, vol. 40, no. 5, 1 October 2007 (2007-10-01), pages 755-767, XP055054093, ISSN: 0960-7722, DOI: 10.1111/j.1365-2184.2007.00468.x

## Description

### BACKGROUND

A common issue in mammalian cell culture is cell death at the end of culture, which can be caused by various insults such as nutrient depletion, inhibitor build-up, and/or oxidative stress, among others. It is of great interest in general, and especially to those who utilize mammalian culture systems to express pharmaceutically relevant protein products, to maintain cell viability and/or delay cell death over the duration of cell culture processes. Several methods have been employed in order to increase viability of cell cultures, such as uses of media additives and overexpression of anti-apoptosis genes. See, for example, Arden, et al. "Chemical caspase inhibitors enhance cell culture viabilities and protein titer" Biotechnol Prog. 2007 Mar-Apr; 23 (2): 506-511; Mastrangelo, et al. "Antiapoptosis chemicals prolong productive lifetimes of mammalian cells upon Sindbis virus vector infection" Biotechnol Bioeng. 1999 Nov 5; 65 (3): 298-305; Balcarcel, et al. "Rapamycin Reduces Hybridoma Cell Death and Enhances Monoclonal Antibody Production" Biotechnology and Bioengineering, 2001 Vol. 76, 1-10; Zanghi et al. "The growth factor inhibitor suramin reduces apoptosis and cell aggregation in protein-free CHO cell batch cultures. "Biotechnol. Prog. 2000, 16, 319-325; Simpson, et al. "Prevention of hybridoma cell death by bcl-2 during suboptimal culture conditions" Biotechnol Bioeng 1997, 54: 1-16; Singh, et al. "Enhancement of survivability of mammalian cells by overexpression of the apoptosis suppressor gene bcl-2." Biotechnol Bioeng. 1996, 52: 166-175; Mastrangelo, et al. "Overexpression of bcl-2 family members enhances survival of mammalian cells in response to various culture insults." Biotechnology and Bioengineering, 2000, Vol 67, 555-564; Arden, et al. "Inhibiting the apoptosis pathway using MDM2 in mammalian cell cultures." Biotechnology and Bioengineering, 2007; Vol. 97, 601-614). However, in some cases, the use of media additives can delay cell cycle progression, resulting in lower cell density, and thus less productivity. Some media additives may protect cells from apoptosis during the growth phase but are not effective during the death phase. Additionally, most media additives are costly, which may not be practical for large-scale pharmaceutical manufacturing. For over expression of anti-apoptosis genes, gene transfection is challenging and the effect maybe case dependent. WO2009/087087 discloses that the addition of iron to cell culture media may improve the viability of cells, the cell density, and cellular product formation.

Therefore, there is a need to improve cell culture for increased cell viability, density and/or titer.

### SUMMARY

The present invention as defined in the appended claims encompasses the unexpected discovery that the addition of iron, in particular, high concentrations of iron to cell culture medium can significantly improve cell density, viability and/or titer of the culture, among other benefits. Thus, the present invention provides an effective yet inexpensive and easy solution for improving cell culture. The present invention is particularly useful for improving viability at the end of extended cell culture.

In one aspect, the present disclosure provides methods of increasing cell density, viability, and/or titer in a cell culture including steps of providing cells in a cell culture medium to start a cell culture process (e.g., a product cell culture process), and adding a composition comprising iron to said cell culture medium during the cell culture process such that the concentration of iron in the cell culture medium is increased over the course of the cell culture process.

According to the present invention, the composition comprising iron may be selected from a variety of iron-containing compounds. In some embodiments the composition containing iron is selected from the group consisting of FeSO₄, Fe-citrate, Fe-transferrin, Fe-chloride, Fe-nitrate, Fe-EDTA, Fe(NO₃)₃, FeCl₂, FeCl₃ and combinations thereof.

In some embodiments, methods in accordance with the present disclosure include addition of a composition comprising iron at a range of time points throughout the cell culture process. In some embodiments, a composition comprising iron is added after day 0. In some embodiments, a composition comprising iron is added on or after day 1. In some embodiments, a composition comprising iron is added on or after day 3. In some embodiments, a composition comprising iron is added on or after day 6. In some embodiments, a composition comprising iron is added on or after day 9. In some embodiments, a composition comprising iron is added at multiple time points during the cell culture process.

In some embodiments, the concentration of iron in the cell culture medium after one or more additions of a composition comprising iron ranges between 100 µM and 5 mM. In some embodiments the concentration of iron in the cell culture medium ranges between 300 µM and 1 mM. In some embodiments, the concentration of iron in the cell culture medium is 1 mM.

A variety of cell types may be used in accordance with the present invention. For example, in some embodiments, the cells are mammalian cells. In some embodiments, the mammalian cells are selected from a BALB/c mouse myeloma line, human retinoblasts (PER.C6), monkey kidney cells, human embryonic kidney line (293), baby hamster kidney cells (BHK), Chinese hamster ovary cells (CHO), mouse sertoli cells, African green monkey kidney cells (VERO-76), human cervical carcinoma cells (HeLa), canine kidney cells, buffalo rat liver cells, human lung cells, human liver cells, mouse mammary tumor cells, TRI cells, MRC 5 cells, FS4 cells, or a human hepatoma line (Hep G2). In some embodiments, the mammalian cells are CHO cells.

In some embodiments, the cell culture process is a fed batch culture process. In some embodiments, the cell culture process is a batch-refeed culture process. In some embodiments, the cell culture process is a perfusion culture process. In some embodiments, the cell culture process is a large-scale production culture process. In some embodiments, the volume of the cell culture medium is at least about 500 L. In some embodiments, the cell culture is carried out in shake flasks.

In some embodiments, supplemental proteins are added to improve cell culture density, viability and/or titer. In some embodiments, the cell culture medium does not contain hydrolysates. In some embodiments, the cell culture medium contains hydrolysates.

In some embodiments, the cells carry a gene that encodes a recombinant protein. In some embodiments, the recombinant protein is an antibody or fragment thereof. In some embodiments, the antibody is an anti-IL-22 antibody. In some embodiments, the antibody is an anti-GDF8 antibody. In some embodiments, the antibody is a Myo29 antibody. In some embodiment, the antibody is a single domain antibody. In some embodiments, the single domain antibody is an anti-TNF nanobody. In some embodiments, the recombinant protein is a glycoprotein. In some embodiments, the recombinant protein is a therapeutic protein. In some embodiments, the therapeutic protein is an antibody, a growth factor, a clotting factor, a cytokine, a fusion protein, a pharmaceutical drug substance, a vaccine, an enzyme, or a Small Modular ImmunoPharmaceutical^{™} (SMIP). In some embodiments, inventive methods described herein further comprise a step of purifying the recombinant protein.

In another aspect, the present disclosure provides a recombinant protein produced from the cells cultured according to the methods described herein.

In yet another aspect, the present disclosure provides methods of preventing or delaying cell death in a cell culture by adding a composition comprising iron at one or more time points subsequent to the beginning of the cell culture process.

In another aspect, the present disclosure provides methods of inhibiting apoptosis in a cell culture by adding a composition comprising iron at one or more time points subsequent to the beginning of the cell culture process.

In some embodiments, the composition comprising iron is added after day 0. In some embodiments, the composition comprising iron is added on or after day 3. In some embodiments, the composition comprising iron is added on or after day 6. In some embodiments, the composition comprising iron is added on or after day 9.

In some embodiments, iron is added in an amount to effect a concentration in the cell culture medium ranging between 100 µM and 5 mM. In some embodiments, iron is added in an amount to effect a concentration in the cell culture medium ranging between 300 µM and 1 mM. In some embodiments, iron is added in an amount to effect a concentration in the cell culture medium of 1 mM.

In some embodiments, the cell culture is a fed batch culture. In some embodiments, the cell culture is a batch-refeed culture. In some embodiments, the cell culture is a perfusion culture. In some embodiments, the cell culture is a large-scale production culture. In some embodiments, the volume of the cell culture is at least about 500 L. In some embodiments, the cell culture is carried out in shake flasks.

In some embodiments, the composition comprising iron is selected from the group consisting of FeSO₄, Fe-citrate, Fe-transferrin, Fe-chloride, Fe-nitrate, Fe-EDTA, Fe(NO₃)₃, FeCl₂, FeCl₃ and combinations thereof.

In yet another aspect, the present disclosure provides, among other things, kits for making a cell culture medium comprising one or more reagents for making an initial cell culture medium, and a separate composition comprising iron. In some embodiments, the kit comprises an instruction to add the separate composition comprising iron to the initial cell culture medium at one or more time points during the course of a cell culture process, e.g., to increase cell density, viability, and/or titer.

In some embodiments, the separate composition comprising iron is in an amount to effect an iron concentration ranging between 100 µM and 5 mM in the initial cell culture medium. In some embodiments, the separate composition is in an amount to effect an iron concentration ranging between 100 µM and 5 mM in a cell culture of least 500L.

In some embodiments, the separate composition is selected from the group consisting of FeSO₄, Fe-citrate, Fe-transferrin, Fe-chloride, Fe-nitrate, Fe-EDTA, Fe(NO₃)₃, FeCl₂, FeCl₃ and combinations thereof.

In some embodiments, the kit further comprises supplementary components for a fed batch culture. In some embodiments, the kit further comprises supplementary components for a batch-refeed culture. In some embodiments, the kit further comprises supplementary components for a perfusion culture. In some embodiments, the supplementary components are selected from the group consisting of hormones and/or other growth factors, inorganic ions, buffers, vitamins, nucleosides or nucleotides, trace elements, amino acids, lipids, glucose or other energy sources, and combinations thereof.

In this application, the use of "or" means "and/or" unless stated otherwise. As used in this application, the term "comprise" and variations of the term, such as "comprising" and "comprises," are not intended to exclude other additives, components, integers or steps. As used herein, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

Other features, objects, and advantages of the present invention are apparent in the detailed description, drawings and claims that follow. It should be understood, however, that the detailed description, the drawings, and the claims, while indicating embodiments of the present invention, are given by way of illustration only, not limitation.

### BRIEF DESCRIPTION OF THE DRAWINGS

The drawings are for illustration purposes only, not for limitation.
*Figure 1*. Exemplary data demonstrating the effect of different doses of iron addition after day 3 on cell growth of CHO cells in shake flasks. Cell growth is measured in terms of viable cell density (VCD)(the total number of viable cells x 10⁶ cells/ml) at a given time point.
*Figure 2**.* Exemplary data demonstrating the effect of different doses of iron addition after day 3 on viability of CHO cells in shake flasks. Viability is measured as a percentage of (viable cells)/(total cells) in culture at a given time point.
*Figure 3**.* Exemplary data demonstrating the effect of different doses of iron addition after day 3 on day 16 viability of CHO cells in shake flasks. Viability is measured as a percentage of (viable cells)/(total cells) in culture at a given time point.
*Figure 4**.* Exemplary data demonstrating the effect of different doses of iron addition after day 3 on antibody titer at day 16 of CHO cells in shake flasks. Antibody titer is measured in grams of antibody/Liter of culture medium. Antibody titer change is measured as a ratio of [antibody]_{with iron}/[antibody] _{without iron} at a given time point.

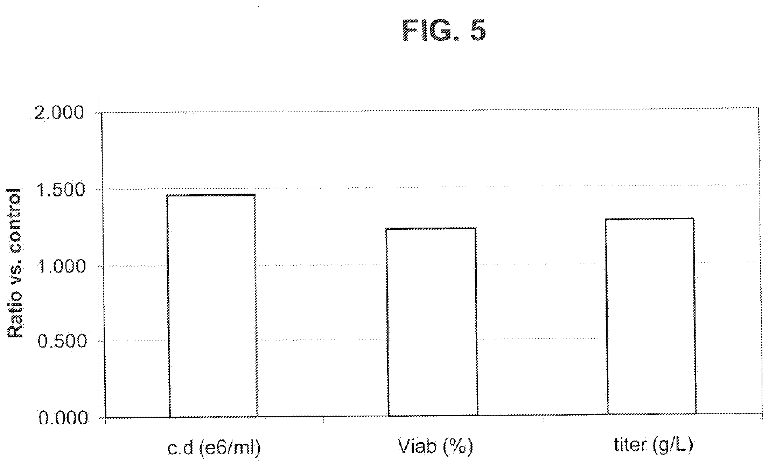

*Figure 5**.* Exemplary data demonstrating the improvement of day 14 cell density, viability, and titer over control upon addition of 1 mM iron to Cell Line 2 culture process. Cell density is measured as the total number of viable cells x 10⁶ cells/ml at a given time point. Cell density improvement is represented as a ratio of cell density_{with iron}/cell density_{without iron}. Viability is measured as a percentage of (viable cells)/(total cells) in culture at a given time point. Viability improvement is measured as a ratio of % viability_{with iron}/% viability_{without iron}. Antibody titer is measured in grams of antibody/Liter of culture medium. Antibody titer change is measured as a ratio of [antibody]_{with iron/}[antibody]_{witho iron} at a given time point.
*Figure 6**.* Exemplary data demonstrating the effect of FeSO₄ or Fe-citrate addition (600 µM) after day 3 on day 14 viability and viable cell density of Cell Line 1 fedbatch culture in shake flasks. Viable cell density is measured as the total number of viable cells x 10⁶ cells/ml at a given time point. Viable cell density improvement is represented as a ratio of cell density_{with iron}/cell density_{without iron}.
*Figure 7*. Exemplary data demonstrating the effect of FeSO₄ addition (1 mM) on day 6 on cell viability of CHO cells producing a nanobody grown in fedbatch culture in shake flasks.
*Figure 8**.* Exemplary data demonstrating the effect of FeSO₄ addition (1 mM) on day 6 on titer of CHO cells producing a nanobody grown in fedbatch culture in shake flasks.
*Figure 9**.* Exemplary data demonstrating the effect of FeSO₄ addition (1 mM) on day 6 on overall productivity of CHO cells producing a nanobody grown in fedbatch culture in shake flasks.
*Figure 10**.* Exemplary data demonstrating the effect of FeSO₄ addition (1 mM) on day 6 on lactate production by CHO cells producing a nanobody grown in fedbatch culture in shake flasks.
*Figure 11**.* Exemplary data demonstrating the effect of FeSO₄ or Fe-citrate addition (600 µM) on cell density of clone 2.8 fedbatch culture in shake flasks.
*Figure 12**.* Exemplary data demonstrating the effect of FeSO₄ or Fe-citrate addition (600 µM) on viability of clone 2.8 fedbatch culture in shake flasks.
*Figure 13**.* Exemplary data demonstrating the effect of FeSO₄ or Fe-citrate addition (600 µM) on overall productivity of clone 2.8 fedbatch culture in shake flasks.
*Figure 14**.* Exemplary data demonstrating the effect of varying doses of day 0 iron addition on day 3 viability of Cell Line 1 in shake flasks. Viability is measured as a percentage of (viable cells)/(total cells) in culture at a given time point.

### DETAILED DESCRIPTION

The present invention as defined in the appended claims provides, among other things, methods and compositions for increasing cell density, viability, and/or titer of product by adding a composition comprising iron at one or more time points over the course of the cell culture process. The present invention as defined in the appended claims encompasses the surprising finding that addition of iron to a cell culture can delay and/or prevent cell death of at the end of a cell culture. In some embodiments, addition of iron to a cell culture can inhibit apoptosis in the cell culture.

Iron is an important component of cell culture medium for growth of mammalian cells. However, prior to the present disclosure, it is thought that low levels of iron may inhibit cell growth upon depletion of iron, while high levels of iron may inhibit cell growth due to toxicity (e.g., oxidative stress and free radical formation). Therefore, it was thought that a balance between the beneficial and toxic qualities of iron is important for mammalian cell culture. Prior to the present invention, conventional media formulations use low concentrations of iron so as to sufficiently support cell growth while remaining below toxic iron concentration levels. As described in the Examples section, the inventors of the present invention have discovered unexpectedly that addition of iron to a cell culture, including iron at concentrations that would be considered high, leads to improved cell growth and delayed cell death of the cell culture resulting in significantly increased cell density, viability and titer, among other benefits. Such effect is independent of cell line, scale, product and Fe source. In addition, the product quality was not affected by the high Fe concentration. Thus, the present disclosure provides a new and cost-effective approach for improved cell culture.

Various aspects of the invention are described in detail in the following sections. The use of sections is not meant to limit the invention. Each section can apply to any aspect of the invention. It is the claims that define the scope of the present invention, which is not and should not be limited to or by this description of certain embodiments.

### Iron Compositions

A wide variety of iron-containing compositions may be used in accordance with the present invention. In certain embodiments, a composition comprising iron is selected from the group comprising FeSO₄, Fe-citrate, Fe-transferrin, Fe-chloride, Fe-nitrate, Fe-EDTA, Fe(NO₃)₃, FeCl₂, FeCl₃ and combinations thereof.

A variety of concentrations of iron may be used in accordance with the present invention. Typically, iron is provided in the medium at a concentration greater than that of a "trace concentration of less than 100µM." The term "trace concentration" as used herein refers to a concentration that may be less than a level ordinarily or easily measured. For example, the trace level of a compound may be <10⁻⁵, <10⁻⁶, <10⁻⁷ or <10⁻⁸ M. In certain embodiments, iron is provided in the medium at a concentration of between approximately 100 µM and 5 mM (e.g., approximately 100 µM - 4.5 mM, 100 µM - 3.0 mM, 100 µM - 2.5 mM, 100 µM - 1.0 mM, 100 µM - 1.0 mM, 200 µM - 2.0 mM, 200 µM - 1.5 mM, 200 µM - 1.0 mM, 150 µM - 4.5 mM, 150 µM - 3.5 mM, 150 µM - 2.5 mM, 150 µM - 1.5 mM, 150 µM - 1.0 mM, 200 µM - 1.5 mM, 200 µM - 1.0 mM, 200 µM - 2.5 mM, 300 µM - 2.5 mM, 300 µM - 1.5 mM, 300 µM - 2.0 mM). In certain embodiments, iron is provided in the medium at a concentration of approximately 100 µM, 150 µM, 200 µM, 250 µM, 300 µM, 350 µM , 400 µM, 450 µM, 500 µM, 550 µM, 600 µM, 650 µM, 700 µM, 750 µM, 800 µM, 850 µM , 900 µM, 950 µM, 1 mM, 1.5 mM, 2 mM, 2.5 mM, 3 mM, 3.5 mM, 4 mM, 4.5 mM, or 5 mM, or at any range within these concentrations. In certain embodiments, iron is provided in the medium at a concentration of between approximately 300 µM and 1mM. In some embodiments, the concentration of iron in a cell culture medium is increased over the course of the cell culture process.

The present disclosure also encompasses the finding that a composition comprising iron may be provided in the medium at any point during the cell culture process. A composition comprising iron may be added to effect the desired iron concentration in the culture medium by adding the composition at one or multiple points over a period of time. In some embodiments, a composition comprising iron is added on or after day 0 of the cell culture process. In some embodiments, a composition comprising iron is added after day 0. In certain embodiments, a composition comprising iron is added on or after day 0, 1, 2, 3, 4, 5, 6, 7, 8, 9, 10, 11, 12, 13, 14, 15, 16, 17, 18, 19, 20, of a cell culture process (e.g., a production cell culture process) or at any combination of the above time points. In some embodiments, a composition comprising iron is added on or after day 3.

One of ordinary skill in the art will be able to choose the exact iron concentration and addition time within these ranges based on the particular attributes of his or her experimental design, including the character of the cells to be cultured, the character of any product (e.g., antibody or recombinant protein) being produced by the cells in culture, the presence or absence of other components in the medium in which the cells are grown, and the growth conditions. For example, cells grown in static culture may be able to more or less optimally utilize iron compared with cells grown in agitated culture (See, e.g., WO 94/02592).

### Cell Culture Media

The terms "medium", "cell culture medium" and "culture medium" as used herein refer to a solution containing nutrients which nourish growing mammalian cells. Typically, such solutions provide essential and non-essential amino acids, vitamins, energy sources, lipids, and trace elements required by the cell for minimal growth and/or survival. Such a solution may also contain supplementary components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), inorganic compounds present at high final concentrations (e.g., iron), amino acids, lipids, and/or glucose or other energy source. In certain embodiments, a medium is advantageously formulated to a pH and salt concentration optimal for cell survival and proliferation. In certain embodiments, a medium is a feed medium that is added after the beginning of the cell culture.

A wide variety of mammalian growth media may be used in accordance with the present invention. In certain embodiments, cells may be grown in one of a variety of chemically defined media, wherein the components of the media are both known and controlled. In certain embodiments, cells may be grown in a complex medium, in which not all components of the medium are known and/or controlled.

Chemically defined growth media for mammalian cell culture have been extensively developed and published over the last several decades. All components of defined media are well characterized, and so defined media do not contain complex additives such as serum or hydrolysates. Early media formulations were developed to permit cell growth and maintenance of viability with little or no concern for protein production. More recently, media formulations have been developed with the express purpose of supporting highly productive recombinant protein producing cell cultures.

Not all components of complex media are well characterized, and so complex media may contain additives such as simple and/or complex carbon sources, simple and/or complex nitrogen sources, and serum, among other things. In some embodiments, complex media suitable for the present invention contains additives such as hydrolysates in addition to other components of defined medium as described herein.

In some embodiments, defined media typically includes roughly fifty chemical entities at known concentrations in water. Most of them also contain one or more well-characterized proteins such as insulin, IGF-1, transferrin or BSA, but others require no protein components and so are referred to as protein-free defined media. Typical chemical components of the media fall into five broad categories: amino acids, vitamins, inorganic salts, trace elements, and a miscellaneous category that defies neat categorization.

Cell culture medium may be optionally supplemented with supplementary components. The term "supplementary components" as used herein refers to components that enhance growth and/or survival above the minimal rate, including, but not limited to, hormones and/or other growth factors, particular ions (such as sodium, chloride, calcium, magnesium, and phosphate), buffers, vitamins, nucleosides or nucleotides, trace elements (inorganic compounds usually present at very low final concentrations), amino acids, lipids, and/or glucose or other energy source. In certain embodiments, supplementary components may be added to the initial cell culture. In certain embodiments, supplementary components may be added after the beginning of the cell culture.

Typically, trace elements refer to a variety of inorganic salts included at micromolar or lower levels. For example, commonly included trace elements are zinc, selenium, copper, and others. In the present invention iron (ferrous or ferric salts) is included as a trace element in the inital cell culture medium at a concentraton of less than 100 µM. As discussed above, the present invention provides methods that include adding iron in addition to the trace amount of less than 100 µM of iron present in the initial cell culture medium such that the iron concentrations in the cell culture medium are greater than trace amount (e.g., greater than 100 µM, 200 µM, 300 µM, 400 µM, 500 µM, 600 µM, 700 µM, 800 µM, 900 µM, or 1 mM). Manganese is also frequently included among the trace elements as a divalent cation (MnCl₂ or MnSO₄) in a range of nanomolar to micromolar concentrations. Numerous less common trace elements are usually added at nanomolar concentrations.

### Cells

Any host cell susceptible to cell culture may be utilized in accordance with the present invention. In certain embodiments, a host cell is mammalian. Non-limiting examples of mammalian cells that may be used in accordance with the present invention include BALB/c mouse myeloma line (NSO/I, ECACC No: 85110503); human retinoblasts (PER.C6, CruCell, Leiden, The Netherlands); monkey kidney CV1 line transformed by SV40 (COS-7, ATCC CRL 1651); human embryonic kidney line (293 or 293 cells subcloned for growth in suspension culture, Graham et al., J. Gen Virol., 36:59,1977); baby hamster kidney cells (BHK, ATCC CCL 10); Chinese hamster ovary cells +/-DHFR (CHO, Urlaub and Chasin, Proc. Natl. Acad. Sci. USA, 77:4216, 1980); mouse sertoli cells (TM4, Mather, Biol. Reprod., 23:243-251, 1980); monkey kidney cells (CV1 ATCC CCL 70); African green monkey kidney cells (VERO-76, ATCC CRL-1 587); human cervical carcinoma cells (HeLa, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci., 383:44-68, 1982); MRC 5 cells; FS4 cells; and a human hepatoma line (Hep G2).

Additionally, any number of commercially and non-commercially available hybridoma cell lines may be utilized in accordance with the present invention. The term "hybridoma" as used herein refers to a cell or progeny of a cell resulting from fusion of an immortalized cell and an antibody-producing cell. Such a resulting hybridoma is an immortalized cell that produces antibodies. Individual cells used to create the hybridoma can be from any mammalian source, including, but not limited to, rat, pig, rabbit, sheep, pig, goat, and human. In certain embodiments, a hybridoma is a trioma cell line, which results when progeny of heterohybrid myeloma fusions, which are the product of a fusion between human cells and a murine myeloma cell line, are subsequently fused with a plasma cell. In certain embodiments, a hybridoma is any immortalized hybrid cell line that produces antibodies such as, for example, quadromas (See, e.g., Milstein et al., Nature, 537:3053, 1983). One skilled in the art will appreciate that hybridoma cell lines might have different nutrition requirements and/or might require different culture conditions for optimal growth, and will be able to modify conditions as needed.

### Cell Culture Methods

The terms "culture" and "cell culture" as used herein refer to a cell population that is suspended in a medium under conditions suitable to survival and/or growth of the cell population. As will be clear to those of ordinary skill in the art, in certain embodiments, these terms as used herein refer to the combination comprising the cell population and the medium in which the population is suspended. In certain embodiments, the cells of the cell culture comprise mammalian cells.

The present invention may be used with any cell culture method that is amenable to the desired process (e.g., production of a recombinant protein (e.g., antibody)). As a non-limiting example, cells may be grown in batch or fed-batch cultures, where the culture is terminated after sufficient expression of the recombinant protein (e.g., antibody), after which the expressed protein (e.g., antibody) is harvested. Alternatively, as another non-limiting example, cells may be grown in batch-refeed or perfusion cultures, where the culture is not terminated and new nutrients and other components are periodically or continuously added to the culture, during which the expressed recombinant protein (e.g., antibody) is harvested periodically or continuously. Other suitable methods (e.g., spin-tube cultures) are known in the art and can be used to practice the present invention.

In certain embodiments, a cell culture suitable for the present invention is a fed-batch culture. The term "fed-batch culture" as used herein refers to a method of culturing cells in which additional components are provided to the culture at a time or times subsequent to the beginning of the culture process. Such provided components typically comprise nutritional components for the cells which have been depleted during the culturing process. Additionally or alternatively, such additional components may include supplementary components, as described herein. In certain embodiments, additional components are provided in a feed medium, as described herein. A fed-batch culture is typically stopped at some point and the cells and/or components in the medium are harvested and optionally purified.

In certain embodiments, a cell culture suitable for the present invention is a batch-refeed culture. The term "batch-refeed culture" as used herein refers to a method of culturing cells in which a portion of the cells (e.g., about 50%, about 55%, about 60%, about 65%, about 70%, about 75%, about 80%, about 85%, about 90%, about 95%, or more of cells) are removed from the cell culture after innoculation and growth for a particular amount of time (e.g., 1 day, 2 days, 3 days, 4 days, 5 days, 6 days, 7 days, etc.). Typically, cell-containing medium that is removed from batch-refeed cultures is replaced with an equivalent volume of fresh medium. In certain embodiments, additional components are provided in a refeed medium, as described herein. A batch refeed culture typically is a continuous process which involves expansion and maintenance of cell cultures within the culture vessel (e.g., bioreactor).

In certain embodiments, a cell culture suitable for the present invention is a perfusion culture. Typically, perfusion culture involves maintenance of a working volume cell culture medium by continuous introduction of fresh culture medium and removal of spent medium via the use of a cell retention system. In some embodiments, cells may be retained in culture using any available method, for example, filtration, sedimentation, centrifugation, and combinations thereof. In some embodiments, perfusion cultures can be grown for extended periods of time (e.g., 2 weeks, 3 weeks, 4 weeks, 5 weeks, or more).

Cells may be grown in any convenient volume chosen by the practitioner. For example, cells may be grown in small scale reaction vessels ranging in volume from a few milliliters to several liters. Alternatively, cells may be grown in large scale commercial Bioreactors ranging in volume from approximately at least 1 liter to 10, 100, 250, 500, 1000, 2500, 5000, 8000, 10,000, 12,000 liters or more, or any volume in between.

The temperature of a cell culture will be selected based primarily on the range of temperatures at which the cell culture remains viable and the range in which a high level of desired product (e.g., a recombinant protein or antibody) is produced. For example, Cell Line 1 grows well and can produce high titer antibody at approximately 37°C. In general, most mammalian cells grow well and can produce desired products (e.g., recombinant proteins or antibodies) within a range of about 25°C to 42°C, although methods taught by the present disclosure are not limited to these temperatures. Certain mammalian cells grow well and can produce desired products (e.g., recombinant proteins or antibodies) within the range of about 35°C to 40°C. In certain embodiments, a cell culture is grown at a temperature of 20, 21, 22, 23, 24, 25, 26, 27, 28, 29, 30, 31, 32, 33, 34, 35, 36, 37, 38, 39, 40, 41, 42, 43, 44, 45°C at one or more times during the cell culture process. Those of ordinary skill in the art will be able to select appropriate temperature or temperatures in which to grow cells, depending on the particular needs of the cells and the particular production requirements of the practitioner. The cells may be grown for any amount of time, depending on the needs of the practitioner and the requirement of the cells themselves.

A culture may be subjected to one or more temperature shifts during the course of the culture. When shifting the temperature of a culture, the temperature shift may be relatively gradual. For example, it may take several hours or days to complete the temperature change. Alternatively, the temperature shift may be relatively abrupt. The temperature may be steadily increased or decreased during the culture process. Alternatively, the temperature may be increased or decreased by discrete amounts at various times during the culture process. The subsequent temperature(s) or temperature range(s) may be lower than or higher than the initial or previous temperature(s) or temperature range(s). One of ordinary skill in the art will understand that multiple discrete temperature shifts are encompassed in this embodiment. For example, the temperature may be shifted once (either to a higher or lower temperature or temperature range), the cells maintained at this temperature or temperature range for a certain period of time, after which the temperature may be shifted again to a new temperature or temperature range, which may be either higher or lower than the temperature or temperature range of the previous temperature or temperature range. The temperature of the culture after each discrete shift may be constant or may be maintained within a certain range of temperatures.

In certain embodiments, the cell density, viability, and/or titer of the cell culture are determined. The term "cell density" as used herein refers to the number of cells present in a given volume of medium. The term "cell viability" as used herein refers to the ability of cells in culture to survive under a given set of culture conditions or experimental variations. The term as used herein also refers to that portion of cells which are alive at a particular time in relation to the total number of cells, living and dead, in the culture at that time. Those of ordinary skill in the art will appreciate that one of many methods for determining cell viability are encompassed in this invention. For example, one may use a dye (e.g., trypan blue) that does not pass through the membrane of a living cell, but can pass through the disrupted membrane of a dead or dying cell in order to determine cell viability. The cell viability of cultures subjected to various culture conditions (e.g., iron concentrations or duration of culture) may be determined and compared to one another to determine optimal growth conditions for such cultures. The term "titer" as used herein refers, for example, to the total amount of recombinantly expressed protein or antibody produced by a mammalian cell culture in a given amount of medium volume. Titer is typically expressed in units of milligrams of protein, e.g., antibody, per milliliter of medium.

In certain embodiments, batch and fed-batch reactions are terminated once the desired cell density, viability, and/or titer is reached, as determined by the needs of the practitioner. As a non-limiting example, the culture may be terminated once the cells reach 1, 5, 10, 15, 20, 25, 30, 35, 40, 45, 50, 55, 60, 65, 70, 75, 80, 85, 90, 95 or 99 percent of maximal viable cell density. Additionally or alternatively, batch and fed-batch reactions may be terminated prior to excessive accumulation of metabolic waste products such as lactate and ammonium. In some embodiments, batch and fed-batch reactions may be terminated once accumulation of waste products (e.g., lactate and/or ammonium) reaches 1, 2, 3, 4, 5, 6, 7, 8, 9, 10 or greater g/L of culture medium.

In certain cases, it may be beneficial to supplement a cell culture during the subsequent production phase with nutrients or other medium components that have been depleted or metabolized by the cells. As non-limiting examples, it may be beneficial to supplement a cell culture with hormones and/or other growth factors, inorganic ions (such as, for example, sodium, chloride, calcium, magnesium, and phosphate), buffers, vitamins, nucleosides or nucleotides, trace elements, inorganic compounds at levels higher than trace concentration (e.g., iron), amino acids, lipids, or glucose or other energy source. Such supplementary components may all be added to the cell culture at one time, or they may be provided to the cell culture in a series of additions.

One of ordinary skill in the art will be able to tailor specific cell culture conditions in order to optimize certain characteristics of the cell culture including but not limited to growth rate, cell viability, final cell density of the cell culture, final concentration of detrimental metabolic byproducts such as lactate and ammonium, final titer of the desired product (e.g., recombinant protein or antibody), or any combination of these or other conditions deemed important by the practitioner.

### Expression of Proteins

As noted above, in many instances the cells will be selected or engineered to produce high levels of desired products (e.g., recombinant protein or antibody). Often, cells will be manipulated by the hand of man to produce high levels of recombinant protein, for example by introduction of a gene encoding the protein of interest and/or by introduction of genetic control elements that regulate expression of that gene (whether endogenous or introduced).

Certain proteins may have detrimental effects on cell growth, cell viability or some other characteristic of the cells that ultimately limits production of the protein of interest in some way. Even amongst a population of cells of one particular type engineered to express a specific protein, variability within the cellular population exists such that certain individual cells will grow better, produce more protein of interest, or produce a protein with higher activity levels (e.g., enzymatic activity). In certain embodiments, a cell line is empirically selected by the practitioner for robust growth under the particular conditions chosen for culturing the cells. In some embodiments, individual cells engineered to express a particular protein are chosen for large-scale production based on cell growth, final cell density, percent cell viability, titer of the expressed protein or any combination of these or any other conditions deemed important by the practitioner.

Any protein that is expressible in a host cell may be produced in accordance with the present teachings. The term "host cell" as used herein refers to a cell that is manipulated according to the present invention to produce a protein of interest as described herein. In some embodiments, a host cell is a mammalian cell. A protein may be expressed from a gene that is endogenous to the host cell, or from a heterologous gene that is introduced into the host cell. A protein may be one that occurs in nature, or may alternatively have a sequence that was engineered or selected by the hand of man.

Proteins that may desirably be expressed in accordance with the present invention will often be selected on the basis of an interesting or useful biological or chemical activity. For example, the present invention may be employed to express any pharmaceutically or commercially relevant enzyme, receptor, antibody, hormone, regulatory factor, antigen, binding agent, etc. In some embodiments, the protein expressed by cells in culture are selected from antibodies, or fragments thereof, nanobodies, single domain antibodies, glycoproteins, therapeutic proteins, growth factors, clotting factors, cytokines, fusion proteins, pharmaceutical drug substances, vaccines, enzymes, or Small Modular ImmunoPharmaceuticals^{™} (SMIPs). The list of proteins that can be produced according to the present invention is merely exemplary in nature, and is not intended to be a limiting recitation. One of ordinary skill in the art will understand that any protein may be expressed in accordance with the present invention and will be able to select the particular protein to be produced based on his or her particular needs.

### Antibodies

Given the large number of antibodies currently in use or under investigation as pharmaceutical or other commercial agents, production of antibodies is of particular interest in accordance with the present invention. Antibodies are proteins that have the ability to specifically bind a particular antigen. Any antibody that can be expressed in a host cell may be produced in accordance with the present invention. In some embodiments, the antibody to be expressed is a monoclonal antibody.

In some embodiments, the monoclonal antibody is a chimeric antibody. A chimeric antibody contains amino acid fragments that are derived from more than one organism. Chimeric antibody molecules can include, for example, an antigen binding domain from an antibody of a mouse, rat, or other species, with human constant regions. A variety of approaches for making chimeric antibodies have been described. See *e.g*., Morrison et al., Proc. Natl. Acad. Sci. U.S.A. 81, 6851 (1985); Takeda et al., Nature 314, 452 (1985), Cabilly et al., U.S. Patent No. 4,816,567; Boss et al., U.S. Patent No. 4,816,397; Tanaguchi *et al*., European Patent Publication EP171496; European Patent Publication 0173494, United Kingdom Patent GB 2177096B.

In some embodiments, the monoclonal antibody is a human antibody derived, e.g., through the use of ribosome-display or phage-display libraries (*see, e.g*., Winter et al., U.S. Patent No. 6,291,159 and Kawasaki, U.S. Patent No. 5,658,754) or the use of xenographic species in which the native antibody genes are inactivated and functionally replaced with human antibody genes, while leaving intact the other components of the native immune system (*see, e.g.,* Kucherlapati et al., U.S. Patent No. 6,657,103).

In some embodiments, the monoclonal antibody is a humanized antibody. A humanized antibody is a chimeric antibody wherein the large majority of the amino acid residues are derived from human antibodies, thus minimizing any potential immune reaction when delivered to a human subject. In humanized antibodies, amino acid residues in the complementarity determining regions are replaced, at least in part, with residues from a non-human species that confer a desired antigen specificity or affinity. Such altered immunoglobulin molecules can be made by any of several techniques known in the art, (*e.g*., Teng et al., Proc. Natl. Acad. Sci. U.S.A., 80, 7308-7312 (1983); Kozbor et al., Immunology Today, 4, 7279 (1983); Olsson et al., Meth. Enzymol., 92, 3-16 (1982)), and are preferably made according to the teachings of PCT Publication WO92/06193 or EP 0239400). Humanized antibodies can be commercially produced by, for example, Scotgen Limited, 2 Holly Road, Twickenham, Middlesex, Great Britain. For further reference, see Jones et al., Nature 321:522-525 (1986); Riechmann et al., Nature 332:323-329 (1988); and Presta, Curr. Op. Struct. Biol. 2:593-596 (1992).

In some embodiments, the monoclonal, chimeric, or humanized antibodies described above may contain amino acid residues that do not naturally occur in any antibody in any species in nature. These foreign residues can be utilized, for example, to confer novel or modified specificity, affinity or effector function on the monoclonal, chimeric or humanized antibody. In some embodiments, the antibodies described above may be conjugated to drugs for systemic pharmacotherapy, such as toxins, low-molecular-weight cytotoxic drugs, biological response modifiers, and radionuclides (see e.g., Kunz et al., Calicheamicin derivative-carrier conjugates, US20040082764 A1).

In some embodiments, the present invention is used to produce an antibody that specifically binds to the Aβ fragment of amyloid precursor protein or to other components of an amyloid plaque, and is useful in combating the accumulation of amyloid plaques in the brain which characterize Alzheimer's disease. (See, e.g., US Provisional Application 60/636,684.) In some embodiments, the present invention is used to produce an antibody that specifically binds the HER2/neu receptor. In some embodiments, the present invention is used to produce an anti-CD20 antibody. In some embodiments, the present invention is used to produce antibodies against TNFα, CD52, CD25, VEGF, EGFR, CD11a, CD33, CD3, IL-22, alpha-4 integrin, and/or IgE.

In another embodiment, antibodies of the present invention are directed against cell surface antigens expressed on target cells and/or tissues in proliferative disorders such as cancer. In one embodiment, the antibody is an IgG1 anti-Lewis Y antibody. Lewis Y is a carbohydrate antigen with the structure Fuc 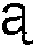 1 → 2Galβ1 → 4[Fuc 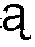 1 → 3]GlcNacβ1 →3R (Abe et al. (1983) J. Biol. Chem., 258 11793-11797). Lewis Y antigen is expressed on the surface of 60% to 90% of human epithelial tumors (including those of the breast, colon, lung, and prostate), at least 40% of which overexpress this antigen, and has limited expression in normal tissues.

In order to target Ley and effectively target a tumor, an antibody with exclusive specificity to the antigen is ideally required. Thus, preferably, the anti-Lewis Y antibodies of the present invention do not cross-react with the type 1 structures (i.e., the lacto-series of blood groups (Lea and Leb)) and, preferably, do not bind other type 2 epitopes (i.e., neolacto-structure) like Lex and H-type 2 structures. An example of a preferred anti-Lewis Y antibody is designated hu3S193 (see U.S. Patent Nos. 6,310,185; 6,518,415; 5,874,060). The humanized antibody hu3S193 (Attia, M.A., et al. 1787-1800) was generated by CDR-grafting from 3S193, which is a murine monoclonal antibody raised against adenocarcinoma cell with exceptional specificity for Ley (Kitamura, K., 12957-12961). Hu3S193 not only retains the specificity of 3S193 for Ley but has also gained in the capability to mediate complement dependent cytotoxicity (hereinafter referred to as CDC) and antibody dependent cellular cytotoxicity (hereinafter referred to as ADCC) (Attia, M.A., et al. 1787-1800). This antibody targets Ley expressing xenografts in nude mice as demonstrated by biodistribution studies with hu3S193 labeled with 125I, 111In, or 18F, as well as other radiolabels that require a chelating agent, such as 111In, 99mTc, or 90Y (Clark, et al. 4804-4811).

In some embodiments, the antibody is one of the human anti-GDF-8 antibodies termed Myo29, Myo28, and Myo22, and antibodies and antigen- binding fragments derived therefrom. These antibodies are capable of binding mature GDF-8 with high affinity, inhibit GDF-8 activity *in vitro* and *in vivo* as demonstrated, for example, by inhibition of ActRIIB binding and reporter gene assays, and may inhibit GDF-8 activity associated with negative regulation of skeletal muscle mass and bone density. *See, e.g.,* Veldman, et al, U.S. Patent Application No. 20040142382.

### Receptors

Another class of polypeptides that have been shown to be effective as pharmaceutical and/or commercial agents includes receptors. Receptors are typically trans-membrane glycoproteins that function by recognizing an extra-cellular signaling ligand. Receptors typically have a protein kinase domain in addition to the ligand recognizing domain, which initiates a signaling pathway by phosphorylating target intracellular molecules upon binding the ligand, leading to developmental or metabolic changes within the cell. In one embodiment, the receptors of interest are modified so as to remove the transmembrane and/or intracellular domain(s), in place of which there may optionally be attached an Ig-domain. In a preferred embodiment, receptors to be produced in accordance with the present invention are receptor tyrosine kinases (RTKs). The RTK family includes receptors that are crucial for a variety of functions numerous cell types (see, e.g., Yarden and Ullrich, Ann. Rev. Biochem. 57:433-478, 1988; Ullrich and Schlessinger, Cell 61:243-254, 199). Non-limiting examples of RTKs include members of the fibroblast growth factor (FGF) receptor family, members of the epidermal growth factor receptor (EGF) family, platelet derived growth factor (PDGF) receptor, tyrosine kinase with immunoglobulin and EGF homology domains-1 (TIE-1) and TIE-2 receptors (Sato et al., Nature 376(6535):70-74 (1995) and c-Met receptor, some of which have been suggested to promote angiogenesis, directly or indirectly (Mustonen and Alitalo, J. Cell Biol. 129:895-898, 1995). Other non-limiting examples of RTK's include fetal liver kinase 1 (FLK-1) (sometimes referred to as kinase insert domain-containing receptor (KDR) (Terman et al., Oncogene 6:1677-83, 1991) or vascular endothelial cell growth factor receptor 2 (VEGFR-2)), fms-like tyrosine kinase-1 (Flt-1) (DeVries et al. Science 255;989-991, 1992; Shibuya et al., Oncogene 5:519-524, 1990), sometimes referred to as vascular endothelial cell growth factor receptor 1 (VEGFR-1), neuropilin-1, endoglin, endosialin, and Axl. Those of ordinary skill in the art will be aware of other receptors that can preferably be expressed in accordance with the present invention.

In some embodiments, tumor necrosis factor inhibitors, in the form of tumor necrosis factor alpha and beta receptors (TNFR-1; EP 417,563 published Mar. 20, 1991; and TNFR-2, EP 417,014 published Mar. 20, 1991) are expressed in accordance with the present invention (for review, see Naismith and Sprang, J Inflamm. 47(1-2):1-7 (1995-96). According to one embodiment, the tumor necrosis factor inhibitor comprises a soluble TNF receptor and preferably a TNFR-Ig. In some embodiments, the preferred TNF inhibitors of the present invention are soluble forms of TNFRI and TNFRII, as well as soluble TNF binding proteins, in another embodiment, the TNFR-Ig fusion is a TNFR:Fc, a term which as used herein refers to "*etanercept*," which is a dimer of two molecules of the extracellular portion of the p75 TNF-α receptor, each molecule consisting of a 235 amino acid Fc portion of human IgG1.

### Growth Factors and Other Signaling Molecules

Another class of polypeptides that have been shown to be effective as pharmaceutical and/or commercial agents includes growth factors and other signaling molecules. Growth factors include glycoproteins that are secreted by cells and bind to and activate receptors on other cells, initiating a metabolic or developmental change in the receptor cell. In one embodiment, the protein of interest is an ActRIIB fusion polypeptide comprising the extracellular domain of the ActRIIB receptor and the Fc portion of an antibody (see, e.g., Wolfman, et al., ActRIIB fusion polypeptides and uses therefor, US2004/0223966 A1). In another embodiment, the growth factor may be a modified GDF-8 pro-peptide (see., e.g., Wolfman, et al., Modified and stabilized GDF propeptides and uses thereof, US2003/0104406 A1). Alternatively, the protein of interest could be a follistatin-domain-containing protein (see, e.g., Hill, et al., GASP1: a follistatin domain containing protein, US 2003/0162714 A1, Hill, et al., GASP1: a follistatin domain containing protein, US 2005/0106154 A1, Hill, et al., Follistatin domain containing proteins, US 2003/0180306 A1).

Non-limiting examples of mammalian growth factors and other signaling molecules include cytokines; epidermal growth factor (EGF); platelet-derived growth factor (PDGF); fibroblast growth factors (FGFs) such as aFGF and bFGF; transforming growth factors (TGFs) such as TGF-alpha and TGF-beta, including TGF-beta 1, TGF-beta 2, TGF-beta 3, TGF-beta 4, or TGF-beta 5; insulin-like growth factor-I and -II (IGF-I and IGF-II); des(1-3) -IGF-I (brain IGF-I), insulin-like growth factor binding proteins; CD proteins such as CD-3, CD-4, CD-8, and CD-19; erythropoietin; osteoinductive factors; immunotoxins; a bone morphogenetic protein (BMP); an interferon such as interferon-alpha, -beta, and -gamma; colony stimulating factors (CSFs), e.g., M-CSF, GM-CSF, and G-CSF; interleukins (TLs), e.g., IL-1 to IL-10; IL-10 superfamily cytokines (e.g., IL-19, IL-20, IL-22, IL-24, IL-26); tumor necrosis factor (TNF) alpha and beta; insulin A-chain; insulin B-chain; proinsulin; follicle stimulating hormone; calcitonin; luteinizing hormone; glucagon; clotting factors such as factor VIIIC, factor IX, tissue factor, and von Willebrands factor; anti-clotting factors such as Protein C; atrial natriuretic factor; lung surfactant; a plasminogen activator, such as urokinase or human urine or tissue-type plasminogen activator (t-PA); bombesin; thrombin, hemopoietic growth factor; enkephalinase; RANTES (regulated on activation normally T-cell expressed and secreted); human macrophage inflammatory protein (MIP-1-alpha); mullerian-inhibiting substance; relaxin A-chain; relaxin B-chain; prorelaxin; mouse gonadotropin-associated peptide; neurotrophic factors such as bone-derived neurotrophic factor (BDNF), neurotrophin-3, -4, -5, or -6 (NT-3, NT-4, NT-5, or NT-6), or a nerve growth factor such as NGF-beta. One of ordinary skill in the art will be aware of other growth factors or signaling molecules that can be expressed in accordance with the present invention.

### G-Protein Coupled Receptors

Another class of polypeptides that have been shown to be effective as pharmaceutical and/or commercial agents includes growth factors and other signaling molecules. G-protein coupled receptors (GPCRs) are proteins that have seven transmembrane domains. Upon binding of a ligand to a GPCR, a signal is transduced within the cell which results in a change in a biological or physiological property of the cell.

GPCRs, along with G-proteins and effectors (intracellular enzymes and channels which are modulated by G-proteins), are the components of a modular signaling system that connects the state of intracellular second messengers to extracellular inputs. These genes and gene-products are potential causative agents of disease.

Specific defects in the rhodopsin gene and the V2 vasopressin receptor gene have been shown to cause various forms of autosomal dominant and autosomal recessive retinitis pigmentosa, nephrogenic diabetes insipidus. These receptors are of critical importance to both the central nervous system and peripheral physiological processes. The GPCR protein superfamily now contains over 250 types of paralogues, receptors that represent variants generated by gene duplications (or other processes), as opposed to orthologues, the same receptor from different species. The superfamily can be broken down into five families: Family I, receptors typified by rhodopsin and the beta2-adrenergic receptor and currently represented by over 200 unique members; Family II, the recently characterized parathyroid hormone/calcitonin/secretin receptor family; Family III, the metabotropic glutamate receptor family in mammals; Family IV, the cAMP receptor family, important in the chemotaxis and development of D. discoideum; and Family V, the fungal mating pheromone receptors such as STE2.

GPCRs include receptors for biogenic amines, for lipid mediators of inflammation, peptide hormones, and sensory signal mediators. The GPCR becomes activated when the receptor binds its extracellular ligand. Conformational changes in the GPCR, which result from the ligand-receptor interaction, affect the binding affinity of a G protein to the GPCR intracellular domains. This enables GTP to bind with enhanced affinity to the G protein.

Activation of the G protein by GTP leads to the interaction of the G protein α subunit with adenylate cyclase or other second messenger molecule generators. This interaction regulates the activity of adenylate cyclase and hence production of a second messenger molecule, cAMP. cAMP regulates phosphorylation and activation of other intracellular proteins. Alternatively, cellular levels of other second messenger molecules, such as cGMP or eicosinoids, may be upregulated or downregulated by the activity of GPCRs. The G protein a subunit is deactivated by hydrolysis of the GTP by GTPase, and the αβ and γ subunits reassociate. The heterotrimeric G protein then dissociates from the adenylate cyclase or other second messenger molecule generator. Activity of GPCR may also be regulated by phosphorylation of the intra- and extracellular domains or loops.

Glutamate receptors form a group of GPCRs that are important in neurotransmission. Glutamate is the major neurotransmitter in the CNS and is believed to have important roles in neuronal plasticity, cognition, memory, learning and some neurological disorders such as epilepsy, stroke, and neurodegeneration (Watson, S. and S. Arkinstall (1994) The G- Protein Linked Receptor Facts Book, Academic Press, San Diego CA, pp. 130-132). These effects of glutamate are mediated by two distinct classes of receptors termed ionotropic and metabotropic. Ionotropic receptors contain an intrinsic cation channel and mediate fast excitatory actions of glutamate. Metabotropic receptors are modulatory, increasing the membrane excitability of neurons by inhibiting calcium dependent potassium conductances and both inhibiting and potentiating excitatory transmission of ionotropic receptors. Metabotropic receptors are classified into five subtypes based on agonist pharmacology and signal transduction pathways and are widely distributed in brain tissues.

The vasoactive intestinal polypeptide (VIP) family is a group of related polypeptides whose actions are also mediated by GPCRs. Key members of this family are VIP itself, secretin, and growth hormone releasing factor (GRF). VIP has a wide profile of physiological actions including relaxation of smooth muscles, stimulation or inhibition of secretion in various tissues, modulation of various immune cell activities. and various excitatory and inhibitory activities in the CNS. Secretin stimulates secretion of enzymes and ions in the pancreas and intestine and is also present in small amounts in the brain. GRF is an important neuroendocrine agent regulating synthesis and release of growth hormone from the anterior pituitary (Watson, S. and S. Arkinstall supra, pp. 278-283).

Following ligand binding to the GPCR, a conformational change is transmitted to the G protein, which causes the α-subunit to exchange a bound GDP molecule for a GTP molecule and to dissociate from the Py-subunits. The GTP-bound form of the α-subunit typically functions as an effector-modulating moiety, leading to the production of second messengers, such as cyclic AMP (e.g., by activation of adenylate cyclase), diacylglycerol or inositol phosphates. Greater than 20 different types of α-subunits are known in man, which associate with a smaller pool of β and γ subunits. Examples of mammalian G proteins include Gi, Go, Gq, Gs and Gt. G proteins are described extensively in Lodish H. et al. Molecular Cell Biology, (Scientific American Books Inc., New York, N.Y., 1995).

GPCRs are a major target for drug action and development. In fact, receptors have led to more than half of the currently known drugs (Drews, Nature Biotechnology, 1996, 14: 1516) and GPCRs represent the most important target for therapeutic intervention with 30% of clinically prescribed drugs either antagonizing or agonizing a GPCR (Milligan, G. and Rees, S., (1999) TIPS, 20: 118-124). This demonstrates that these receptors have an established, proven history as therapeutic targets.

In general, practitioners of the present invention will selected their polypeptide of interest, and will know its precise amino acid sequence. Any given protein that is to be expressed in accordance with the present invention will have its own idiosyncratic characteristics and may influence the cell density or viability of the cultured cells, and may be expressed at lower levels than another polypeptide or protein grown under identical culture conditions. One of ordinary skill in the art will be able to appropriately modify the steps and compositions of the present invention in order to optimize cell growth and/or production of any given expressed polypeptide or protein.

### Enzymes

Another class of proteins that have been shown to be effective as pharmaceutical and/or commercial agents includes enzymes. Enzymes may be proteins whose enzymatic activity may be affected by cell culture conditions under which they were produced. Thus, production of enzymes with desirable enzymatic activity in accordance with the present invention is also of particular interest. One of ordinary skill in the art will be aware of many known enzymes that may be expressed by cells in culture.

Non-limiting examples of enzymes include a carbohydrase, such as an amylase, a cellulase, a dextranase, a glucosidase, a galactosidase, a glucoamylase, a hemicellulase, a pentosanase, a xylanase, an invertase, a lactase, a naringanase, a pectinase and a pullulanase; a protease such as an acid protease, an alkali protease, bromelain, ficin, a neutral protease, papain, pepsin, a peptidase (e.g., an aminopeptidase and carboxypeptidase), rennet, rennin, chymosin, subtilisin, thermolysin, an aspartic proteinase, and trypsin; a lipase or esterase, such as a triglyceridase, a phospholipase, a pregastric esterase, a phosphatase, a phytase, an amidase, an iminoacylase, a glutaminase, a lysozyme, and a penicillin acylase; an isomerase such as glucose isomerase; an oxidoreductases, such as an amino acid oxidase, a catalase, a chloroperoxidase, a glucose oxidase, a hydroxysteroid dehydrogenase or a peroxidase; a lyase such as a acetolactate decarboxylase, an aspartic decarboxylase, a fumarase or a histadase; a transferase such as cyclodextrin glycosyltranferase; a ligase; a chitinase, a cutinase, a deoxyribonuclease, a laccase, a mannosidase, a mutanase, a pectinolytic enzyme, a polyphenoloxidase, ribonuclease and transglutaminase.

In general, practitioners of the present invention will select a protein of interest, and will know its precise amino acid sequence. Any given protein that is to be expressed in accordance with the present invention may have its own particular characteristics and may influence the cell density or viability of the cultured cells, may be expressed at lower levels than another protein grown under identical culture conditions, and may have different biological activity depending on the exact culture conditions and steps performed. One of ordinary skill in the art will be able to appropriately modify the steps and compositions used to produce a particular protein according to the teachings of the present invention in order to optimize cell growth and the production and/or activity level of any given expressed protein.

### Introduction of Genes for the Expression of Proteins into Host Cells

Generally, a nucleic acid molecule introduced into the cell encodes the protein desired to be expressed according to the present invention. Alternatively, a nucleic acid molecule may encode a gene product that induces the expression of the desired protein by the cell. For example, introduced genetic material may encode a transcription factor that activates transcription of an endogenous or heterologous protein. Alternatively or additionally, an introduced nucleic acid molecule may increase the translation or stability of a protein expressed by the cell.

Methods suitable for introducing nucleic acids sufficient to achieve expression of a protein of interest into mammalian host cells are known in the art. See, for example, Gething et al., Nature, 293:620-625, 1981; Mantei et al., Nature, 281:40-46, 1979; Levinson et al. EP 117,060; and EP 117,058. For mammalian cells, common methods of introducing genetic material into mammalian cells include the calcium phosphate precipitation method of Graham and van der Erb (Virology, 52:456-457, 1978) or the lipofectamine^{™} (Gibco BRL) Method of Hawley-Nelson (Focus 15:73, 1993). General aspects of mammalian cell host system transformations have been described by Axel in U.S. Pat. No. 4,399,216 issued Aug. 16, 1983. For various techniques for introducing genetic material into mammalian cells, see Keown et al., Methods in Enzymology, 1989, Keown et al., Methods in Enzymology, 185:527-537, 1990, and Mansour et al., Nature, 336:348-352, 1988.

In some embodiments, a nucleic acid to be introduced is in the form of a naked nucleic acid molecule. For example, the nucleic acid molecule introduced into a cell may consist only of the nucleic acid encoding the protein and the necessary genetic control elements. Alternatively, a nucleic acid encoding the protein (including the necessary regulatory elements) may be contained within a plasmid vector. Non-limiting representative examples of suitable vectors for expression of proteins in mammalian cells include pCDNA1; pCD, see Okayama, et al. Mol. Cell Biol. 5:1136-1142, 1985; pMCIneo Poly-A, see Thomas, et al. Cell 51:503-512, 1987; a baculovirus vector such as pAC 373 or pAC 610; CDM8 , see Seed, B. Nature 329:840, 1987; and pMT2PC, see Kaufman, et al. EMBO J. 6:187-195, 1987. In some embodiments, a nucleic acid molecule to be introduced into a cell is contained within a viral vector. For example, a nucleic acid encoding the protein may be inserted into the viral genome (or a partial viral genome). Regulatory elements directing the expression of the protein may be included with the nucleic acid inserted into the viral genome (i.e., linked to the gene inserted into the viral genome) or can be provided by the viral genome itself.

Naked DNA can be introduced into cells by forming a precipitate containing the DNA and calcium phosphate. Alternatively, naked DNA can also be introduced into cells by forming a mixture of the DNA and DEAE-dextran and incubating the mixture with the cells or by incubating the cells and the DNA together in an appropriate buffer and subjecting the cells to a high-voltage electric pulse (e.g., by electroporation). A further method for introducing naked DNA cells is by mixing the DNA with a liposome suspension containing cationic lipids. The DNA/liposome complex is then incubated with cells. Naked DNA can also be directly injected into cells by, for example, microinjection.

Alternatively, naked DNA can also be introduced into cells by complexing the DNA to a cation, such as polylysine, which is coupled to a ligand for a cell-surface receptor (see for example Wu, G. and Wu, C.H. J. Biol. Chem. 263:14621,1988; Wilson et al. J. Biol. Chem. 267:963-967, 1992; and U.S. Patent No. 5,166,320,). Binding of the DNA-ligand complex to the receptor facilitates uptake of the DNA by receptor-mediated endocytosis.

Use of viral vectors containing particular nucleic acid sequences, e.g., a cDNA encoding a protein, is a common approach for introducing nucleic acid sequences into a cell. Infection of cells with a viral vector has the advantage that a large proportion of cells receive the nucleic acid, which can obviate the need for selection of cells which have received the nucleic acid. Additionally, molecules encoded within the viral vector, e.g., by a cDNA contained in the viral vector, are generally expressed efficiently in cells that have taken up viral vector nucleic acid.

Defective retroviruses are well characterized for use in gene transfer for gene therapy purposes (for a review see Miller, A.D. Blood 76:271, 1990). A recombinant retrovirus can be constructed having a nucleic acid encoding a protein of interest inserted into the retroviral genome. Additionally, portions of the retroviral genome can be removed to render the retrovirus replication defective. Such a replication defective retrovirus is then packaged into virions which can be used to infect a target cell through the use of a helper virus by standard techniques.

The genome of an adenovirus can be manipulated such that it encodes and expresses a protein of interest but is inactivated in terms of its ability to replicate in a normal lytic viral life cycle. See, for example, Berkner et al. BioTechniques 6:616, 1988; Rosenfeld etal. Science 252:431-434, 1991; and Rosenfeld et al. Cell 68:143-155, 1992. Suitable adenoviral vectors derived from the adenovirus strain Ad type 5 dl324 or other strains of adenovirus (e.g., Ad2, Ad3, Ad7 etc.) are known to those skilled in the art. Recombinant adenoviruses are advantageous in that they do not require dividing cells to be effective gene delivery vehicles and can be used to infect a wide variety of cell types, including airway epithelium (Rosenfeld *et al*., 1992, cited supra), endothelial cells (Lemarchand et al., Proc. Natl. Acad. Sci. USA 89:6482-6486, 1992), hepatocytes (Herz and Gerard, Proc. Natl. Acad. Sci. USA 90:2812-2816, 1993) and muscle cells (Quantin et al., Proc. Natl. Acad. Sci. USA 89:2581-2584, 1992). Additionally, introduced adenoviral DNA (and foreign DNA contained therein) is not integrated into the genome of a host cell but remains episomal, thereby avoiding potential problems that can occur as a result of insertional mutagenesis in situations where introduced DNA becomes integrated into the host genome (e.g., retroviral DNA). Moreover, the carrying capacity of the adenoviral genome for foreign DNA is large (up to 8 kilobases) relative to other gene delivery vectors (Berkner *et al.* cited supra; Haj-Ahmand and Graham, J. Virol. 57:267, 1986). Most replication-defective adenoviral vectors currently in use are deleted for all or parts of the viral E1 and E3 genes but retain as much as 80% of the adenoviral genetic material.

Adeno-associated virus (AAV) is a naturally occurring defective virus that requires another virus, such as an adenovirus or a herpes virus, as a helper virus for efficient replication and a productive life cycle. (For a review see Muzyczka et al. Curr. Topics in Micro. and Immunol., 158:97-129, 1992). It is also one of the few viruses that may integrate its DNA into non-dividing cells, and exhibits a high frequency of stable integration (see for example Flotte et al., Am. J. Respir. Cell. Mol. Biol. 7:349-356, 1992; Samulski et al., J. Virol. 63:3822-3828, 1989; and McLaughlin et al., J. Virol. 62:1963-1973, 1989). Vectors containing as little as 300 base pairs of AAV can be packaged and can integrate. Space for exogenous DNA is limited to about 4.5 kb. An AAV vector such as that described in Tratschin et al. (Mol. Cell. Biol. 5:3251-3260, 1985) can be used to introduce DNA into cells. A variety of nucleic acids have been introduced into different cell types using AAV vectors (see for example Hermonat et al., Proc. Natl. Acad. Sci. USA 81:6466-6470, 1984; Tratschin et al., Mol. Cell. Biol. 4:2072-2081, 1985; Wondisford et al., Mol. Endocrinol. 2:32-39, 1988; Tratschin et al., J. Virol. 51:611-619, 1984; and Flotte et al., J. Biol. Chem. 268:3781-3790, 1993).

When the method used to introduce nucleic acid molecules into a population of cells results in modification of a large proportion of the cells and efficient expression of the protein by the cells, the modified population of cells may be used without further isolation or subcloning of individual cells within the population. That is, there may be sufficient production of the protein by the population of cells such that no further cell isolation is needed and the population can be immediately be used to seed a cell culture for the production of the protein. Alternatively, it may be desirable to isolate and expand a homogenous population of cells from a few cells or a single cell that efficiently produce(s) the protein.

Alternative to introducing a nucleic acid molecule into a cell that encodes a protein of interest, the introduced nucleic acid may encode another polypeptide or protein that induces or increases the level of expression of the protein produced endogenously by a cell. For example, a cell may be capable of expressing a particular protein but may fail to do so without additional treatment of the cell. Similarly, the cell may express insufficient amounts of the protein for the desired purpose. Thus, an agent that stimulates expression of the protein of interest can be used to induce or increase expression of that protein by the cell. For example, the introduced nucleic acid molecule may encode a transcription factor that activates or upregulates transcription of the protein of interest. Expression of such a transcription factor in turn leads to expression, or more robust expression of the protein of interest.

In certain embodiments, a nucleic acid that directs expression of the protein is stably introduced into the host cell. In certain embodiments, a nucleic acid that directs expression of the protein is transiently introduced into the host cell. One of ordinary skill in the art will be able to choose whether to stably or transiently introduce a nucleic acid into the cell based on his or her experimental needs.

A gene encoding a protein of interest may optionally be linked to one or more regulatory genetic control elements. In certain embodiments, a genetic control element directs constitutive expression of the protein. In certain embodiments, a genetic control element that provides inducible expression of a gene encoding the protein of interest can be used. The use of an inducible genetic control element (e.g., an inducible promoter) allows for modulation of the production of the protein in the cell. Non-limiting examples of potentially useful inducible genetic control elements for use in eukaryotic cells include hormone- regulated elements (e.g., see Mader, S. and White, J.H., Proc. Natl. Acad. Sci. USA 90:5603-5607, 1993), synthetic ligand-regulated elements (see, e.g. Spencer, D.M. et al., Science 262:1019-1024, 1993) and ionizing radiation-regulated elements (e.g., see Manome, Y. et al., Biochemistry 32:10607-10613, 1993; Datta, R. et al., Proc. Natl. Acad. Sci. USA 89:10149-10153, 1992). Additional cell-specific or other regulatory systems known in the art may be used in accordance with the invention.

One of ordinary skill in the art will be able to choose and, optionally, to appropriately modify the method of introducing genes that cause the cell to express the protein of interest in accordance with the teachings of the present invention.

### Isolation of the Expressed Protein

In general, it will typically be desirable to isolate and/or purify proteins expressed according to the present invention. In certain embodiments, the expressed protein is secreted into the medium and thus cells and other solids may be removed, as by centrifugation or filtering for example, as a first step in the purification process.

Alternatively, the expressed protein may be bound to the surface of the host cell. For example, the media may be removed and the host cells expressing the protein are lysed as a first step in the purification process. Lysis of mammalian host cells can be achieved by any number of means well known to those of ordinary skill in the art, including physical disruption by glass beads and exposure to high pH conditions.

The expressed protein may be isolated and purified by standard methods including, but not limited to, chromatography (e.g., ion exchange, affinity, size exclusion, and hydroxyapatite chromatography), gel filtration, centrifugation, or differential solubility, ethanol precipitation and/or by any other available technique for the purification of proteins (See, e.g., Scopes, Protein Purification Principles and Practice 2nd Edition, Springer-Verlag, New York, 1987; Higgins, S.J. and Hames, B.D. (eds.), Protein Expression : A Practical Approach, Oxford Univ Press, 1999; and Deutscher, M.P., Simon, M.I., Abelson, J.N. (eds.), Guide to Protein Purification : Methods in Enzymology (Methods in Enzymology Series, Vol. 182), Academic Press, 1997). For immunoaffinity chromatography in particular, the protein may be isolated by binding it to an affinity column comprising antibodies that were raised against that protein and were affixed to a stationary support. Alternatively, affinity tags such as an influenza coat sequence, poly-histidine, or glutathione-S-transferase can be attached to the protein by standard recombinant techniques to allow for easy purification by passage over the appropriate affinity column. Protease inhibitors such as phenyl methyl sulfonyl fluoride (PMSF), leupeptin, pepstatin or aprotinin may be added at any or all stages in order to reduce or eliminate degradation of the protein during the purification process. Protease inhibitors are particularly advantageous when cells must be lysed in order to isolate and purify the expressed protein.

One of ordinary skill in the art will appreciate that the exact purification technique will vary depending on the character of the protein to be purified, the character of the cells from which the protein is expressed, and/or the composition of the medium in which the cells were grown.

### Pharmaceutical Formulations

In certain preferred embodiments of the invention, produced polypeptides or proteins will have pharmacologic activity and will be useful in the preparation of pharmaceuticals. Compositions as described above may be administered to a subject or may first be formulated for delivery by any available route including, but not limited to parenteral (e.g., intravenous), intradermal, subcutaneous, oral, nasal, bronchial, opthalmic, transdermal (topical), transmucosal, rectal, and vaginal routes. Inventive pharmaceutical compositions typically include a purified polypeptide or protein expressed from a mammalian cell line, a delivery agent (i.e., a cationic polymer, peptide molecular transporter, surfactant, etc., as described above) in combination with a pharmaceutically acceptable carrier. As used herein the language "pharmaceutically acceptable carrier" includes solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use typically include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, NJ) or phosphate buffered saline (PBS). In all cases, the composition should be sterile and should be fluid to the extent that easy syringability exists. Preferred pharmaceutical formulations are stable under the conditions of manufacture and storage and must be preserved against the contaminating action of microorganisms such as bacteria and fungi. In general, the relevant carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, or sodium chloride in the composition. Prolonged absorption of the injectable compositions can be brought about by including in the composition an agent which delays absorption, for example, aluminum monostearate and gelatin.

Sterile injectable solutions can be prepared by incorporating the purified polypeptide or protein in the required amount in an appropriate solvent with one or a combination of ingredients enumerated above, as required, followed by filtered sterilization. Generally, dispersions are prepared by incorporating the purified polypeptide or protein expressed from a mammalian cell line into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. For the purpose of oral therapeutic administration, the purified polypeptide or protein can be incorporated with excipients and used in the form of tablets, troches, or capsules, *e.g*., gelatin capsules. Oral compositions can also be prepared using a fluid carrier for use as a mouthwash. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. Formulations for oral delivery may advantageously incorporate agents to improve stability within the gastrointestinal tract and/or to enhance absorption.

For administration by inhalation, the compositions comprising a purified polypeptide or protein expressed from a mammalian cell line and a delivery agent are preferably delivered in the form of an aerosol spray from a pressured container or dispenser which contains a suitable propellant, *e.g*., a gas such as carbon dioxide, or a nebulizer. The present disclosure particularly contemplates delivery of the compositions using a nasal spray, inhaler, or other direct delivery to the upper and/or lower airway. Intranasal administration of DNA vaccines directed against influenza viruses has been shown to induce CD8 T cell responses, indicating that at least some cells in the respiratory tract can take up DNA when delivered by this route, and the delivery agents of the disclosure will enhance cellular uptake. According to certain embodiments of the disclosure the compositions comprising a purified polypeptide expressed from a mammalian cell line and a delivery agent are formulated as large porous particles for aerosol administration.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the purified polypeptide or protein and delivery agents are formulated into ointments, salves, gels, or creams as generally known in the art.

The compositions can also be prepared in the form of suppositories (*e.g*., with conventional suppository bases such as cocoa butter and other glycerides) or retention enemas for rectal delivery.

In one embodiment, the compositions are prepared with carriers that will protect the polypeptide or protein against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

It is advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active polypeptide or protein calculated to produce the desired therapeutic effect in association with the required pharmaceutical carrier.

The polypeptide or protein expressed according to the present invention can be administered at various intervals and over different periods of time as required, e.g., one time per week for between about 1 to 10 weeks, between 2 to 8 weeks, between about 3 to 7 weeks, about 4, 5, or 6 weeks, etc. The skilled artisan will appreciate that certain factors can influence the dosage and timing required to effectively treat a subject, including but not limited to the severity of the disease or disorder, previous treatments, the general health and/or age of the subject, and other diseases present. Generally, treatment of a subject with a polypeptide or protein as described herein can include a single treatment or, in many cases, can include a series of treatments. It is furthermore understood that appropriate doses may depend upon the potency of the polypeptide or protein and may optionally be tailored to the particular recipient, for example, through administration of increasing doses until a preselected desired response is achieved. It is understood that the specific dose level for any particular animal subject may depend upon a variety of factors including the activity of the specific polypeptide or protein employed, the age, body weight, general health, gender, and diet of the subject, the time of administration, the route of administration, the rate of excretion, any drug combination, and the degree of expression or activity to be modulated.

The present disclosure includes the use of compositions for treatment of nonhuman animals. Accordingly, doses and methods of administration may be selected in accordance with known principles of veterinary pharmacology and medicine. Guidance may be found, for example, in Adams, R. (ed.), Veterinary Pharmacology and Therapeutics, 8th edition, Iowa State University Press; ISBN: 0813817439; 2001.

Pharmaceutical compositions can be included in a container, pack, or dispenser together with instructions for administration.

### Kits

The present disclosure also provides kits including components useful in making a cell culture medium including an composition comprising iron as described herein. Such kits may be of particular use for increasing cell density, viability, and/or titer of a cell culture.

In some embodiments, such kits include one or more reagents for making an initial cell culture medium. Such kits may include one or more reagents useful in supplementing a defined cell culture medium (e.g., hormones and/or other growth factors; inorganic ions such as sodium, chloride, calcium, magnesium, and phosphate; buffers; vitamins; nucleosides or nucleotides; trace elements; amino acids; lipids; or glucose or other energy source). Kits may include a separate composition comprising iron (e.g. FeSO₄, Fe-citrate, Fe-transferrin, Fe-chloride, Fe-nitrate, Fe-EDTA, Fe(NO₃)₃, FeCl₂, or FeCl₃). In some embodiments, kits include a separate composition comprising iron which will effect suitable concentrations in the cell culture medium described herein (e.g., ranging between 100 µM and 5 mM). Kits may also contain instructions (e.g., user's manual) to add the separate composition of iron to the initial cell culture medium at one or more time points during the course of a cell culture process described herein.

Components of kits may provided in individual containers and multiple such containers may be provided together in a common housing.

The present invention is illustrated in further details by the following non-limiting examples. The examples are provided for illustration only and should not be construed as limiting the scope of the invention.

### EXAMPLES

### Example 1: Addition of Fe to Fed Batch Culture After Day 3

This experiment demonstrates that addition of Fe during fed batch culture of cells producing therapeutic proteins after day 3 results in significant increase in the cell density, viability and titer.

CHO cells, Cell Line 1, were cultured in a chemically defined enriched medium using a fed batch culture process. A high concentration of Fe (e.g., 100 µM to 5 mM) was added to the cell culture after day 3 to effect different Fe concentrations (e.g., 0 mM, 0.1 mM, 1.0 mM, 2.0 mM, or 5.0 mM). In this experiment, ferric citrate was used and the cells were cultured in shake flasks. Cells were subjected to cell density, viability, and titer analysis at various time points throughout the cell culture process.

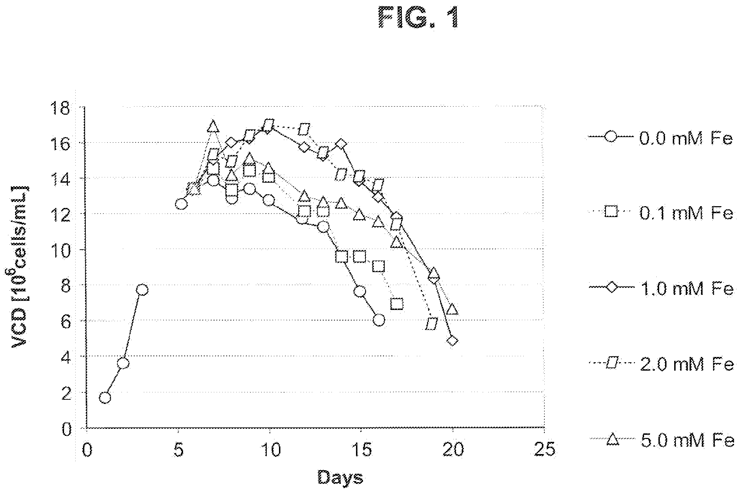

Viable cell density was determined by CEDEX using a digital image recognition method. The effect of different doses of Fe addition after day 3 on cell growth of exemplary Cell Line 1 is shown in Figure 1. As can be seen from Figure 1, the addition of Fe after day 3 increased viable cell density as compared to cells grown in the absence of iron.

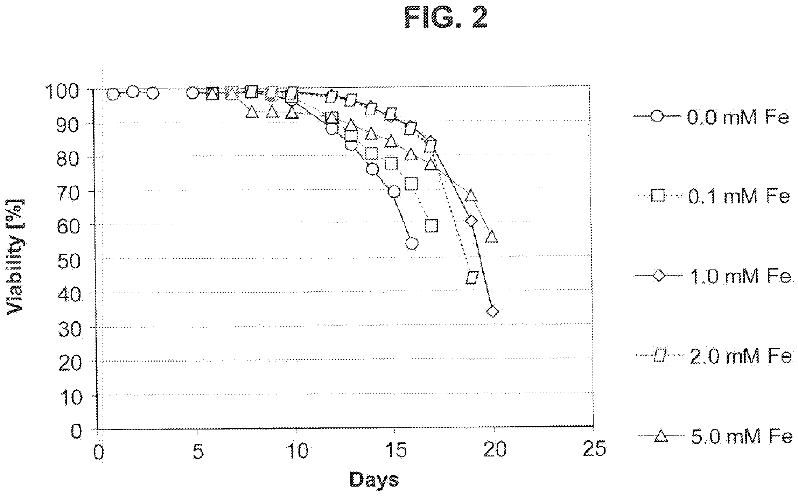

Cell viability was determined CEDEX by trypan blue exclusion method. The effect of different doses of Fe addition after day 3 on cell viability of exemplary Cell Line 1 is shown in Figure 2. As shown in Figure 2, the addition of Fe after day 3 increased cell viability as compared to cells grown in the absence of iron.

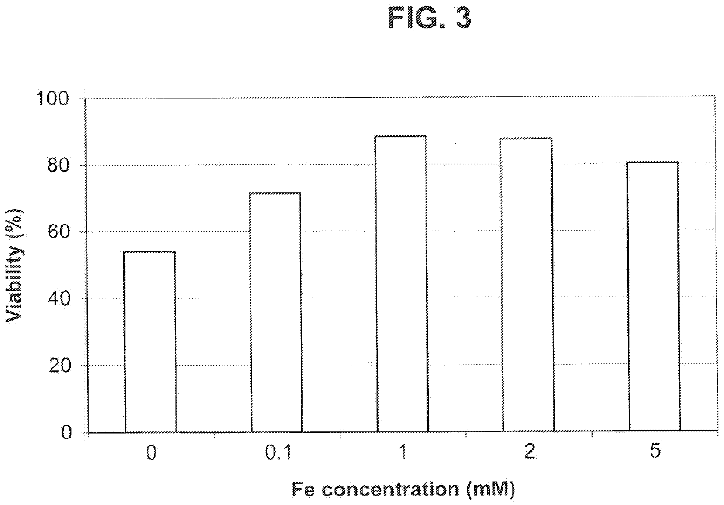

In addition, the effect of Fe addition on the viability of cells at the late stage of cell culture was determined. Specifically, cell viability was determined on day 16 for cells grown in the presence or absence of iron. As shown in Figure 3, addition of Fe increased the cell viability at day 16.

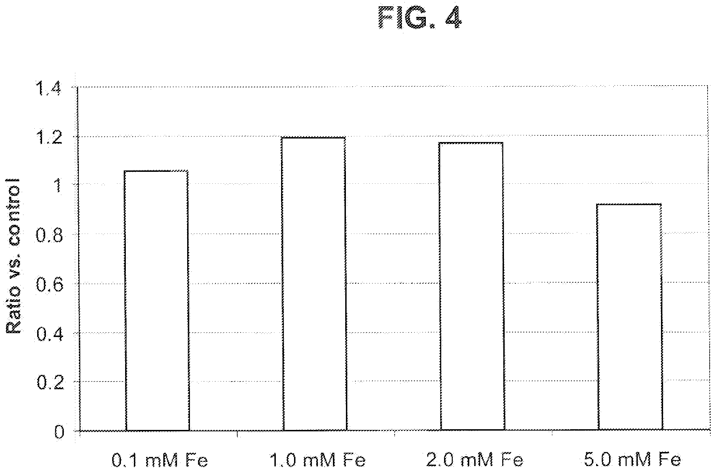

The effect of different doses of Fe addition after day 3 on antibody titer was also determined. The antibody titer was determined by protein A affinity HPLC methods on day 16 for cells grown in the presence or absence of varying concentrations of iron. As shown in Figure 4, addition of Fe increased the antibody titer at day 16.

It was also observed that the optimum post day 3 Fe addition concentration is likely to be from 0.3 mM to 1 mM. Fe addition on any day after day 3 is effective. Distributing Fe over multiple feeds can be better than one lump addition.

Similar experiments were done on a second exemplary cell line, Cell Line 2, and exemplary results are shown in Figure 5. As shown in Figure 5, the addition of Fe improved cell density, viability and titer, especially at the late stage.

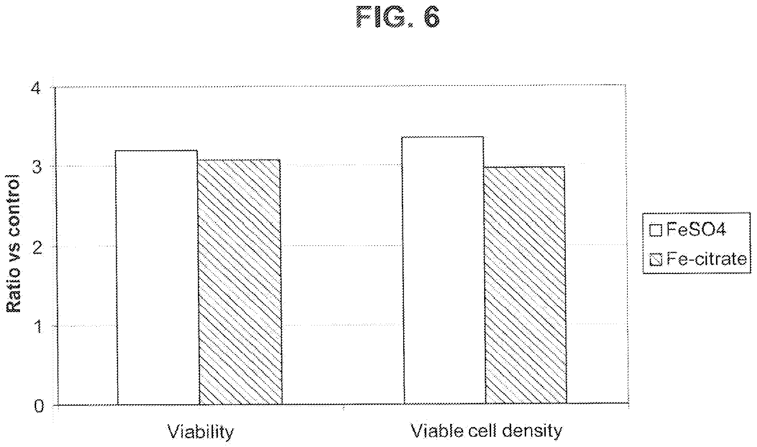

In addition to Fe-citrate, the effect of other Fe complexes such as FeSO₄ or Fe-transferrin was also tested. Figure 6 summarizes exemplary results showing the effect of FeSO₄ and Fe-citrate addition after day 3 on viable cell density and viability on day 14 of Cell Line 1 of fed batch culture.

Similar experiments have also been done in both shake flask and bioreactor experiments and the effect was the same. This effect has been observed for different products (such as different monoclonal antibodies).

Thus, the beneficial effect of Fe addition is independent of cell line, cell culture scale, Fe source and product. It was also determined that the product quality was not affected by the high Fe concentration.

### Example 2: Addition of Fe on Day 6

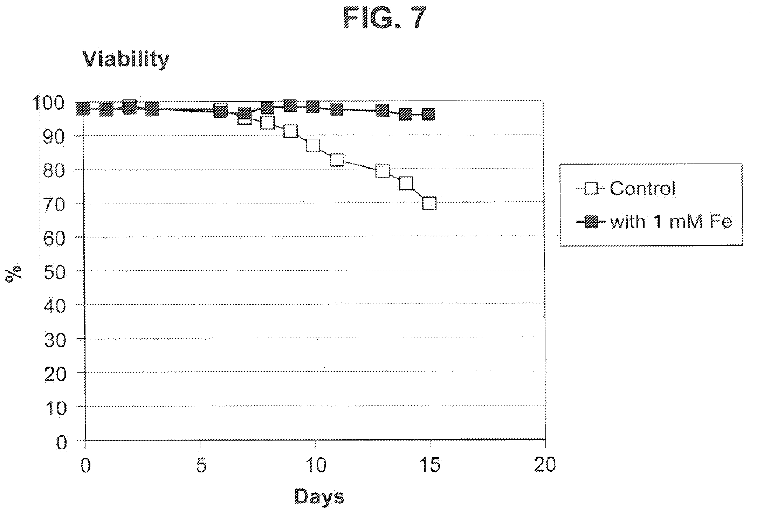

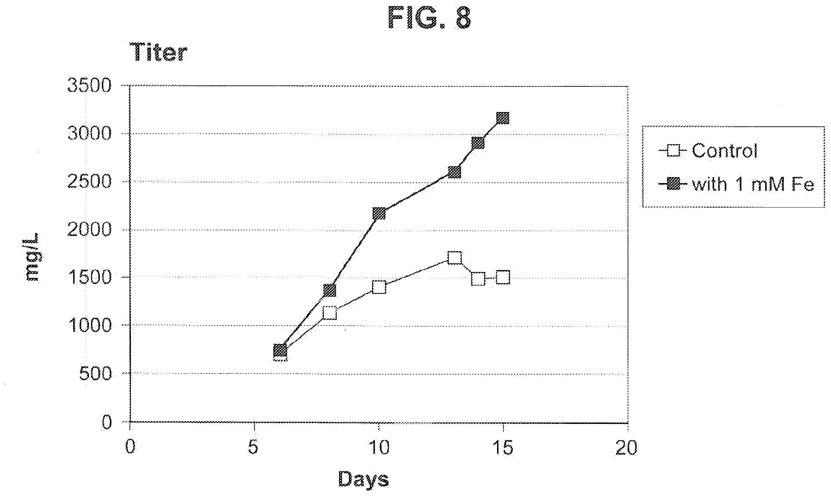

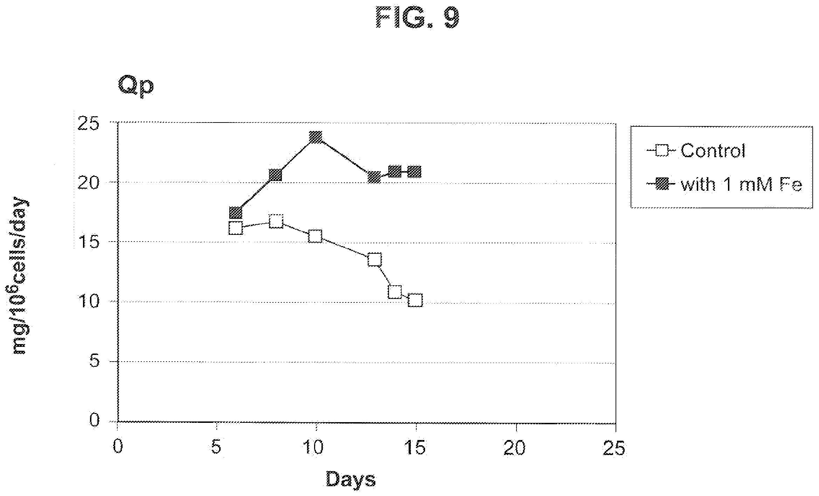

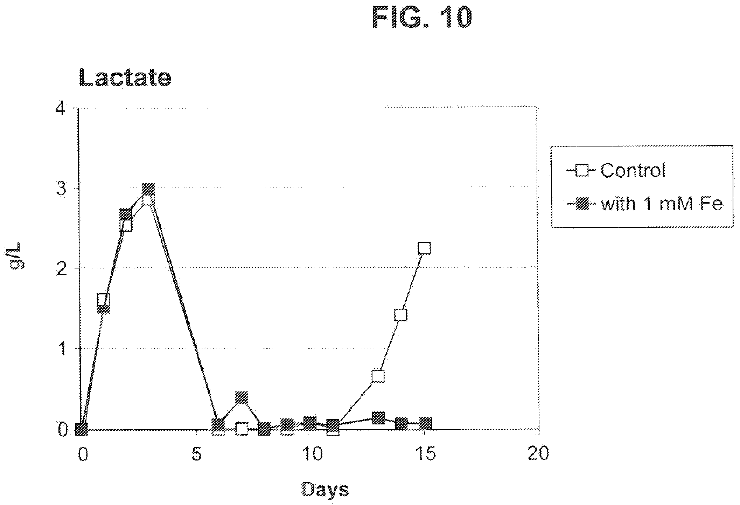

CHO cells expressing an nanobody were grown by fed batch culture in shake flasks. Cells were initially cultured in 7% CO₂ incubator at 37 °C for 0-4 days and shifted to 31 °C after day 4. FeSO₄ was added on day 6 to effect a concentration of 1 mM of iron in the cell culture medium. Cells were subjected to cell density, viability, titer, lactate level and Qp analysis at various time points throughout the cell culture process as described in Example 1. Exemplary results are shown in Figures 7 to 10. As shown in Figures 7 to 10, Fe addition showed significant benefit during cell culture including viability improvement (e.g., 96% vs. 70% on day 15 as shown in Figure 7), titer improvement (e.g., 3.2 g/L vs. 1.5 g/L on day 15 as shown in Figure 8), Qp improvement (e.g., 20 µg/e6/day vs. 10 µg/e6/day as shown in Figure 9), and reduction of lactate level (e.g., lactate remained close to 0 cell culture with Fe addition, but not for control culture without Fe addition, which increased to 2.24 g/L on day 15, as shown in Figure 10).

### Example 3: Fe addition at day 0

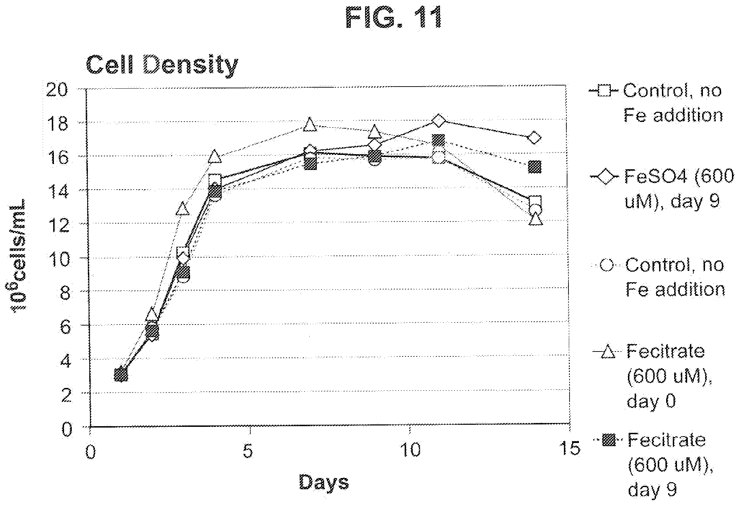

This experiment is designed to test whether a high concentration of Fe added at day 0 would have an advantageous effect. Cells of a cell line producing a monoclonal antibody (clone 2.8) were cultured in pH-controlled shake flasks with 7% CO₂ at -120 rpm using an orbital shaker. Seed density was 1.5 X 10⁶ cells/mL. The cells were cultured for 14 days. The basal medium was a chemically defined basal medium supplemented with amino acids including 4 mM glutamine. Feed medium is a chemically defined concentrated feed medium. Fe-citrate or FeSO₄ was added on day 0 or day 9 to effect a concentration of 600 µM of iron in the cell culture medium. Cells were subjected to cell density, viability, titer and Qp analysis at various time points throughout the cell culture process as described in previous examples. Exemplary results are shown in Figures 11 to 13.

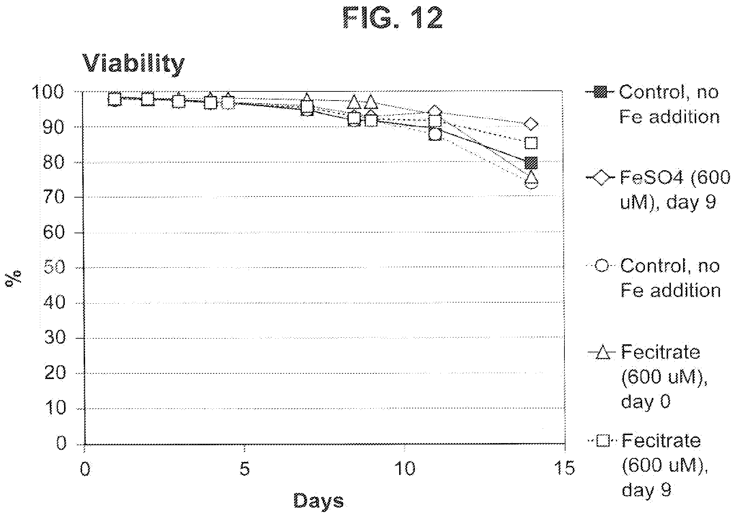

Addition of Fe-citrate at day 0 lead to increased cell growth at early stage of cell culture, while Fe addition on day 9 showed improved cell density at later stage of cell culture (Figure 11). However, Fe-citrate addition on day 0 does not appear to help maintain higher viability at the end of cell culture. For example, as shown in Figure 12, the viability on day 14 for cell culture with Fe-citrate addition at 600 µM on day 0 is about 75%, which is comparable to the 74-80% viability on day 14 for control culture without Fe addition. By contrast, the cell viability for cell culture with Fe addition on day 9 is 85-90%.

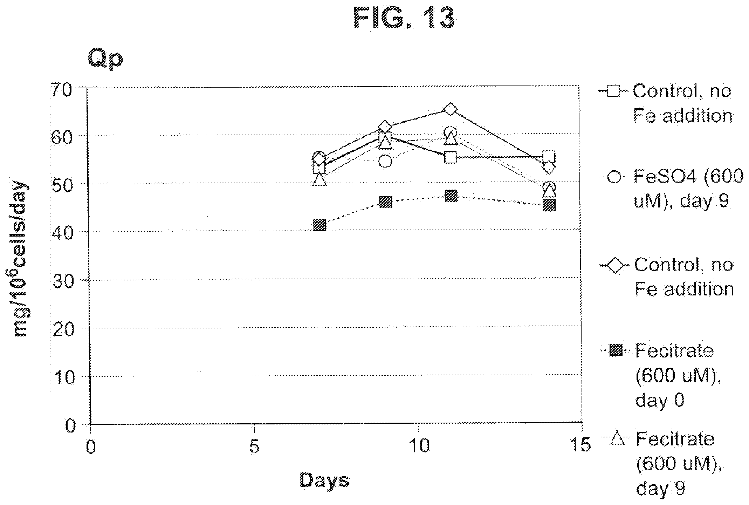

In addition, as shown in Figure 13, the Qp of cell culture with Fe-citrate addition on day 0 is lower than all other conditions throughout the culture.

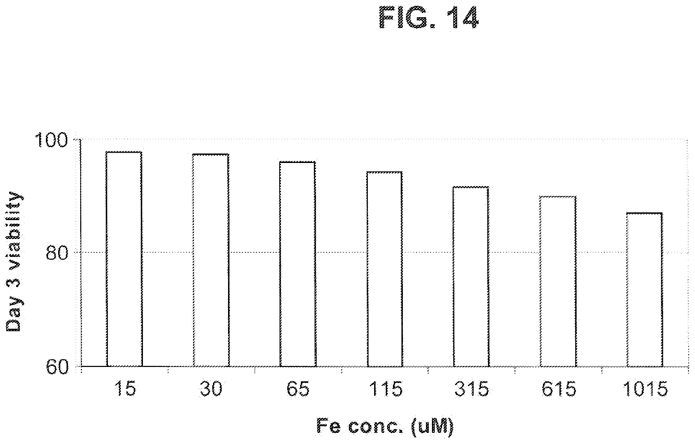

In some cases, when Fe was added at the beginning of the cell culture, toxicity was observed at high Fe concentrations. For example, Fe was added to Cell Line 1 on day 0 and exemplary effects on cell viability on day 3 in shake flasks are shown in Figure 14. As can be seen from Figure 14, toxicity was observed for Fe concentrations higher than 100 µM. Compared to the results shown in Examples 1 and 2, it is surprising that high concentrations of Fe added after day 0 not only had no toxicity, but also demonstrated a benefit to cell culture including cell viability and cell growth.

### EQUIVALENTS

Those skilled in the art will recognize, or be able to ascertain using no more than routine experimentation, many equivalents to the specific embodiments of the invention, described herein. The scope of the present invention is not intended to be limited to the above Description, but rather is as set forth in the appended claims.

Publications discussed above and throughout the text are provided solely for their disclosure prior to the filing date of the present application.

## Claims

1. A method of increasing cell density, viability, and/or titer in a cell culture medium comprising steps of:
(a) providing cells in a cell culture medium to start a cell culture process wherein said cell culture medium comprises iron as a trace element at a concentration of less than 100µM; and
(b) adding a composition comprising iron to said cell culture medium during the cell culture process such that the concentration of iron in the cell culture medium is increased over the course of the cell culture process,
wherein the composition comprising iron is added on or after day 3 of the cell culture process,
wherein the concentration of iron in the cell culture medium after addition of the composition comprising iron ranges between 100 µM and 5 mM,.

2. The method of claim 1, wherein the composition comprising iron is selected from the group consisting of FeSO₄, Fe-citrate, Fe-transferrin, Fe-chloride, Fe-nitrate, Fe-EDTA, Fe(NO3)3, FeCl2, FeCl3 and combinations thereof.

3. The method of claim 1, wherein the composition comprising iron is added on or after day 6 of the cell culture process.

4. The method of claim 1, wherein the composition comprising iron is added at multiple time points during the cell culture process.

5. The method of claim 1, wherein the concentration of iron in the cell culture medium after addition of the composition comprising iron ranges between 300 µM and 1 mM.

6. The method of claim 1, wherein the cells are mammalian cells.

7. The method of claim 6, wherein the mammalian cells are CHO cells.

8. The method of claim 1, wherein the cell culture process is a large-scale production culture process.

9. The method of claim 8, wherein the volume of the cell culture medium is at least about 500 L.

10. The method of claim 1, wherein the cells carry a gene that encodes a recombinant protein.

11. The method of claim 10, wherein the recombinant protein is an antibody or fragment thereof.

12. The method of claim 10, wherein the recombinant protein is a therapeutic protein.

13. The method of any one of claims 10-12, wherein the method further comprises purifying the recombinant protein.

## Patentansprüche

1. Verfahren zur Steigerung von Zelldichte, Zellviabilität und/oder Zelltiter in einem Zellkulturmedium, umfassend die folgenden Schritte:
(a) Bereitstellen von Zellen in einem Zellkulturmedium zum Start eines Zellkultivierungsprozesses, wobei das Zellkulturmedium Eisen als Spurenelement in einer Konzentration von weniger als 100 µM umfasst; sowie
(b) Hinzufügen einer Zusammensetzung, welche Eisen umfasst, zu dem Zellkulturmedium während des Zellkultivierungsprozesses, so dass die Konzentration von Eisen in dem Zellkulturmedium über den Verlauf des Zellkultivierungsprozesses gesteigert wird,
wobei die Zusammensetzung, welche Eisen umfasst, während oder nach Tag 3 des Zellkultivierungsprozesses hinzugefügt wird,
wobei die Konzentration von Eisen in dem Zellkulturmedium nach Hinzufügen der Zusammensetzung, welche Eisen umfasst, zwischen 100 µM und 5 mM liegt.

2. Verfahren gemäß Anspruch 1, wobei die Zusammensetzung, welche Eisen umfasst, ausgewählt ist aus der Gruppe, bestehend aus FeSO₄, Fe-Citrat, Fe-Transferrin, Fe-Chlorid, Fe-Nitrat, Fe-EDTA, Fe(NO₃)₃, FeCl₂, FeCl₃ sowie Kombinationen davon.

3. Verfahren gemäß Anspruch 1, wobei die Zusammensetzung, welche Eisen umfasst, an oder nach Tag 6 des Zellkultivierungsprozesses hinzugefügt wird.

4. Verfahren gemäß Anspruch 1, wobei die Zusammensetzung, welche Eisen umfasst, an mehreren Zeitpunkten während des Zellkultivierungsprozesses zugegeben wird.

5. Verfahren gemäß Anspruch 1, wobei die Konzentration von Eisen in dem Zellkulturmedium nach Hinzufügen der Zusammensetzung, welche Eisen umfasst, zwischen 300 µM und 1 mM liegt.

6. Verfahren gemäß Anspruch 1, wobei die Zellen Säugerzellen sind.

7. Verfahren gemäß Anspruch 6, wobei die Säugerzellen CHO-Zellen sind.

8. Verfahren gemäß Anspruch 1, wobei der Zellkultivierungsprozess ein Produktionskultivierungsprozess im großen Maßstab ist.

9. Verfahren gemäß Anspruch 8, wobei das Volumen des Zellkulturmediums etwas mindestens 500 l beträgt.

10. Verfahren gemäß Anspruch 1, wobei die Zellen ein Gen tragen, das für ein rekombinantes Protein codiert.

11. Verfahren gemäß Anspruch 10, wobei das rekombinante Protein ein Antikörper oder Fragment davon ist.

12. Verfahren gemäß Anspruch 10, wobei das rekombinante Protein ein therapeutisches Protein ist.

13. Verfahren gemäß einem der Ansprüche 10-12, wobei das Verfahren des Weiteren das Aufreinigen des rekombinanten Proteins umfasst.

## Revendications

1. Procédé d'augmentation de la densité, de la viabilité et/ou du titre des cellules dans un milieu de culture cellulaire comprenant les étapes de :
(a) fourniture de cellules dans un milieu de culture cellulaire pour démarrer un processus de culture cellulaire dans lequel ledit milieu de culture cellulaire comprend du fer en tant qu'oligoélément à une concentration inférieure à 100 µM ; et
(b) ajout d'une composition comprenant du fer audit milieu de culture cellulaire au cours du processus de culture cellulaire de sorte que la concentration en fer dans le milieu de culture cellulaire soit augmentée au cours du processus de culture cellulaire,
dans lequel la composition comprenant du fer est ajoutée le 3^{e} jour ou après le 3^{e} jour du processus de culture cellulaire,
dans lequel la concentration en fer dans le milieu de culture cellulaire après ajout de la composition comprenant du fer se situe entre 100 µM et 5 mM.

2. Procédé selon la revendication 1, dans lequel la composition comprenant du fer est choisie dans le groupe constitué par FeSO₄, le citrate de F, la Fe-transferrine, le chlorure de Fe, le nitrate de Fe, le Fe-EDTA, Fe(NO3)3, FeCl2, FeCl3 et les combinaisons de ceux-ci.

3. Procédé selon la revendication 1, dans lequel la composition comprenant du fer est ajoutée le 6^{e} jour ou après le 6^{e} jour du processus de culture cellulaire.

4. Procédé selon la revendication 1, dans lequel la composition comprenant du fer est ajoutée à plusieurs moments au cours du processus de culture cellulaire.

5. Procédé selon la revendication 1, dans lequel la concentration en fer dans le milieu de culture cellulaire après ajout de la composition comprenant du fer se situe entre 300 µM et 1 mM.

6. Procédé selon la revendication 1, dans lequel les cellules sont des cellules de mammifère.

7. Procédé selon la revendication 6, dans lequel les cellules de mammifère sont des cellules CHO.

8. Procédé selon la revendication 1, dans lequel le processus de culture cellulaire est un processus de culture à production à grande échelle.

9. Procédé selon la revendication 8, dans lequel le volume du milieu de culture cellulaire est d'au moins environ 500 l.

10. Procédé selon la revendication 1, dans lequel les cellules portent un gène qui code pour une protéine recombinante.

11. Procédé selon la revendication 10, dans lequel la protéine recombinante est un anticorps ou un fragment de celui-ci.

12. Procédé selon la revendication 10, dans lequel la protéine recombinante est une protéine thérapeutique.

13. Procédé selon l'une des revendications 10 à 12, dans lequel le procédé comprend en outre la purification de la protéine recombinante.
